# EUROPEAN PATENT SPECIFICATION

(11) **EP 3 416 672 B1**
(45) Date of publication and mention of the grant of the patent: **23.10.2024**
(21) Application number: 17753910.3
(22) Date of filing: 17.02.2017
(51) Int. Cl.: A61K 38/14, A61K 47/40, A61P 31/04

(54) **ORITAVANCIN FORMULATIONS**
ORITAVANCIN-FORMULIERUNGEN
FORMULATIONS D'ORITAVANCINE

(30) Priority: 18.02.2016 US 201662296989 P
(43) Date of publication of application: 26.12.2018
(73) Proprietor: Melinta Therapeutics, LLC, Parsippany, NJ 07054 (US)
(72) Inventor: GRIFFITH, David, C., Parsippany NJ 07054 (US); FAR, Adel, Rafai, Parsippany NJ 07054 (US); LEHOUX, Dario, Parsippany NJ 07054 (US); KRISHNA, Gopal, Parsippany NJ 07054 (US)
(74) Representative: Dehns
(86) International application number: PCT/US2017/018340
(87) International publication number: WO 2017/143169

(56) References cited:
- EP-B1- 1 278 549
- WO-A1-2004/110473
- WO-A1-2009/036121
- WO-A2-2010/025438
- WO-A2-2010/025438
- WO-A2-2010/129233
- WO-A2-2011/019839
- WO-A2-2014/176068
- WO-A2-2015/031313
- US-A1- 2004 229 775
- US-A1- 2005 026 819
- HONG DENG ET AL: "Pharmacokinetics of Oritavancin Formulated in Polyethylene Glycol or Hydroxypropylcyclodextrin in Hamster Cecal Contents", ABSTRACTS BOOK, INTERSCIENCE CONFERENCE ON ANTIMICROBIAL AGENTS & CHEMOTHERAPY (ICAAC), AMERICAN SOCIETY FOR MICROBIOLOGY, US, vol. 50, 1 January 2010 (2010-01-01), pages A1 - 1361, XP009512300, ISSN: 0733-6373
- DENG, H. ET AL.: "Pharmacokinetics of Oritavancin Formulated in Polyethylene Glycol or Hydroxypropylcyclodextrin in Hamster Cecal Contents", ABSTRACTS OF THE 50TH INTERSCIENCE CONFERENCE ON ANTIMICROBIAL AGENTS AND CHEMOTHERAPY (ICAAC, vol. 50, 2010, pages Al-1361, XP009512300, ISSN: 0733-6373

## Description

### BACKGROUND

### Field of the Invention

The present invention relates to the fields of chemistry and medicine. More particularly, the present invention relates to compositions including oritavancin, their preparation, and their use as therapeutic agents.

### Description of the Related Art

Oritavancin is a novel semi-synthetic, lipoglycopeptide antibiotic. Oritavancin has proven effective in the treatment of adult patients with acute bacterial skin and skin structure infections (ABSSSIs) caused or suspected to be caused by susceptible isolates of designated Gram-positive microorganisms. An extensive pre-approval clinical development program has been conducted. Oritavancin has been well tolerated in studies.

Oritavancin has been approved by the Food and Drug Administration (FDA) for the treatment of adult subjects with acute bacterial skin and skin structure infections caused or suspected to be caused by susceptible isolates of designated Gram-positive microorganisms including methicillin resistant Staphylococcus aureus (MRSA) [ORBACTIV^{™} Package Insert 2014]. Kmeid and Kanafani (Core Evidence, 2015, 19, p39-47) provides a review of the use of oritavancin for treating ABSSSIs. However, current formulations of oritavancin require injecting large volumes of diluent into a subject, and long infusion times in intravenous administration. Thus, the current formulation may be problematic if administered to certain patient populations, for example, renally impaired patients.

WO2004/110473 teaches the use of polyene macrolide antifungal agents in combination with glycopeptide antibacterial agents which may be used for treating fungal infections. Optionally, cyclodextrin compounds may be included in such compositions. WO2009/036121 teaches methods of inhibiting *Clostridium difficile* by administration of glycopeptide antibiotics such as oritavancin. The use of carriers and excipients, such as cyclodextrin derivatives, is also contemplated. Deng et al (Abstracts book, Interscience Conference on Antimicrobial Agents and Chemotherapy (ICAAC), Am. Soc. Microbiol., 2010, Vol. 50, page A1-1361, XP009512300) teaches oral formulations of oritavancin and PEG or hydroxypropyl cyclodextrin.

### SUMMARY OF THE INVENTION

Some embodiments as defined by the claims include a pharmaceutical composition comprising oritavancin, or a salt thereof, and a modified β-cyclodextrin which is a hydroxypropyl β-cyclodextrin in a ratio as described herein.

Other embodiments as defined by the claims include a pharmaceutical composition comprising oritavancin, or a salt thereof, and the modified β-cyclodextrin in a ratio as described herein for use in a method of treating a bacterial infection.

In particular, the present invention provides a pharmaceutical composition comprising:
oritavancin, or a salt thereof, and hydroxypropyl β-cyclodextrin (HPCD);
wherein the HPCD and oritavancin, or salt thereof, are present in a mass ratio of about 0.25:1 to about 4:1, wherein said composition is
   a) in solid form which on reconstitution provides an intravenous pharmaceutical composition; or
   b) in a solution, which is optionally diluted, which provides an intravenous pharmaceutical composition.

The invention also provides a pharmaceutical composition comprising oritavancin, or a salt thereof, and hydroxypropyl β-cyclodextrin (HPCD) for use in a method of treating a bacterial infection comprising:
administering to a subject in need thereof a therapeutically effective amount of a pharmaceutical composition comprising oritavancin, or a salt thereof, HPCD, and an aqueous medium;
wherein the administration is intravenous;
wherein the HPCD and the oritavancin, or salt thereof, are present in the composition in a mass ratio of about 0.25: 1 to about 4:1.

Embodiments of the invention are as set out in the appended claims.

### BRIEF DESCRIPTION OF THE DRAWINGS

**Figure 1** depicts pharmacokinetic data for various oritavancin formulations according to Example 2.
**Figure 2** depicts comparative pharmacokinetic data for various oritavancin formulations according to Example 3.

### DETAILED DESCRIPTION

Oritavancin is a glycopeptide antibiotic that has following structure:

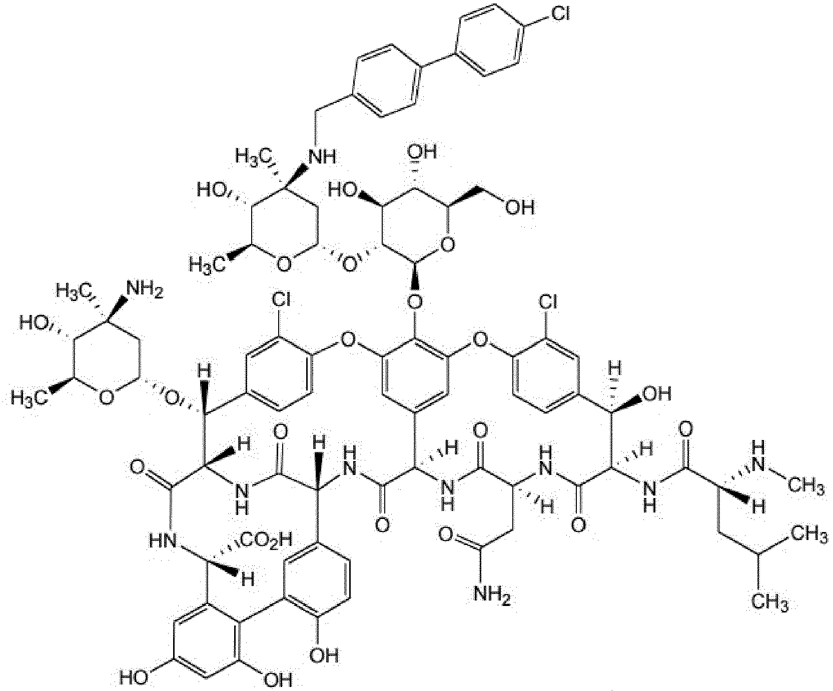

Cyclodextrins are cyclic oligosaccharides consisting of 6, 7, or 8 glucopyranose units, usually referred to as α-, β-, or γ-cyclodextrins, respectively. These compounds have doughnut-shaped structures. They exhibit intramolecular hydrogen bonding between the C2- and C3-hydroxyl groups of neighboring glucopyranose units. Cyclodextrins generally take on the shape of a torus with the C2- and C3-hydroxyls located around the larger opening and the C6-hydroxyl aligned around the smaller opening. The arrangement of C6-hydroxyls opposite the hydrogen bonded C2- and C3-hydroxyls forces the oxygen bonds into close proximity within the cavity, leading to an electron rich, hydrophobic interior. The size of this hydrophobic cavity is a function of the number of glucopyranose units forming the cyclodextrin. The interaction between a cyclodextrin and another molecule may be termed a host-guest relationship, and the complex may be referred to as an inclusion compound or clathrate.

Where the compounds disclosed herein have at least one chiral center, they may exist as individual enantiomers and diastereomers or as mixtures of such isomers, including racemates. Separation of the individual isomers or selective synthesis of the individual isomers is accomplished by application of various methods which are well known to practitioners in the art. Unless otherwise indicated, all such isomers and mixtures thereof are included in the scope of the compounds disclosed herein. Furthermore, compounds disclosed herein may exist in one or more crystalline or amorphous forms. Unless otherwise indicated, all such forms are included in the scope of the compounds disclosed herein including any polymorphic forms. In addition, some of the compounds disclosed herein may form solvates with water (i.e., hydrates) or common organic solvents. Unless otherwise indicated, such solvates are included in the scope of the compounds disclosed herein.

The skilled artisan will recognize that some structures described herein may be resonance forms or tautomers of compounds that may be fairly represented by other chemical structures, even when kinetically; the artisan recognizes that such structures may only represent a very small portion of a sample of such compound(s). Such compounds are considered within the scope of the structures depicted, though such resonance forms or tautomers are not represented herein.

Isotopes may be present in the compounds described. Each chemical element as represented in a compound structure may include any isotope of said element. For example, in a compound structure a hydrogen atom may be explicitly disclosed or understood to be present in the compound. At any position of the compound that a hydrogen atom may be present, the hydrogen atom can be any isotope of hydrogen, including but not limited to hydrogen-1 (protium) and hydrogen-2 (deuterium). Thus, reference herein to a compound encompasses all potential isotopic forms unless the context clearly dictates otherwise.

In some embodiments, the compounds described herein may convert to or exist in equilibrium with alternate forms. Accordingly, in some embodiments, the compounds described herein may exist in combination with one or more of these forms.

### Definitions

Unless defined otherwise, all technical and scientific terms used herein have the same meaning as is commonly understood by one of ordinary skill in the art to which this disclosure belongs. In the event that there is a plurality of definitions for a term herein, those in this section prevail unless stated otherwise.

The term "oritavancin" as provided herein is intended to include all solvates and amorphous, crystalline or polymorphic forms. As provided herein, oritavancin may be present as the free base, or may be present as a salt, for example, an acid addition salt. In some embodiments, oritavancin may be present as the diphosphate salt. In some embodiments, oritavancin may be present as the free base. In some embodiments, oritavancin may be present as a mixture of salt addition states.

"Solvate" refers to the compound formed by the interaction of a solvent and a compound described herein or salt thereof. Suitable solvates are pharmaceutically acceptable solvates including hydrates.

The term "modified β-cyclodextrin" refers to a β-cyclodextrin molecule containing one or more substituent groups or in which one or more chemical moiety has been replaced with another chemical moiety. The term includes derivatized or substituted β-cyclodextrins, and salts thereof. As provided herein, the modified β-cyclodextrin includes β-cyclodextrins modified by any conventional chemical or enzymatic process, for example alkylation, condensation, esterification, etherification, coupling, elimination, intra- or intermolecular cyclization, rearrangement, oxidation, reduction, disproportionation, etc.

The modified β-cyclodextrin for use in pharmaceutical compositions or uses of the invention is a hydroxy propyl β-cyclodextrin.

In general, any modified β-cyclodextrin which is a hydroxy propyl β-cyclodextrin and is suitable for the materials and methods provided herein may be used.. The modified β-cyclodextrin according to the invention is hydroxypropyl β-cyclodextrin ("HPBCD," "HPCD," or "HPβCD").

As provided herein, a single molecule of the modified β-cyclodextrin may include any combination of substituent groups provided herein, and/or different molecules in the modified β-cyclodextrin may include different substitution patterns.

Some β-cyclodextrin derivatives are described, for example, in U.S. Patent Nos. 4,727,064 and 5,376,645 and in U.S. Patent Publication No. 2015/0045311.

As provided herein, the modified β-cyclodextrin may be characterized by a degree of substitution which may be measured, for example, by FTIR spectroscopy. In some embodiments, the modified β-cyclodextrin may be characterized by a degree of substitution from about 2-9. *See* Easton C.J. et al., Modified Cyclodextrins Scaffolds and Templates for Supramolecular Chemistry (1999), World Scientific. The degree of substitution may be an average degree of substitution of all modified β-cyclodextrin molecules in a composition.

The term "pharmaceutically acceptable salt" refers to salts that retain the biological effectiveness and properties of a compound and, which are not biologically or otherwise undesirable for use in a pharmaceutical. In many cases, the compounds disclosed herein are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable salts can also be formed using inorganic and organic bases. Inorganic bases from which salts can be derived include, for example, bases that contain sodium, potassium, lithium, ammonium, calcium, magnesium, iron, zinc, copper, manganese, aluminum, and the like; particularly preferred are the ammonium, potassium, sodium, calcium and magnesium salts. In some embodiments, treatment of the compounds disclosed herein with an inorganic base results in loss of a labile hydrogen from the compound to afford the salt form including an inorganic cation such as Li⁺, Na⁺, K⁺, Mg²⁺ and Ca²⁺ and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Many such salts are known in the art, as described in WO 87/05297, Johnston et al., published September 11, 1987.

As used herein, "kit" means a collection of at least two components constituting the kit. Together, the components constitute a functional unit for a given purpose. Individual member components may be physically packaged together or separately. For example, a kit comprising an instruction for using the kit may or may not physically include the instruction with other individual member components. Instead, the instruction can be supplied as a separate member component, either in a paper form or an electronic form which may be supplied on computer readable memory device or downloaded from an internet website, or as recorded presentation.

As used herein, "instruction(s)" means documents describing relevant materials or methodologies pertaining to a kit. These materials may include any combination of the following: background information, list of components and their availability information (purchase information, etc.), brief or detailed protocols for using the kit, trouble-shooting, references, technical support, and any other related documents. Instructions can be supplied with the kit or as a separate member component, either as a paper form or an electronic form which may be supplied on computer readable memory device or downloaded from an internet website, or as recorded presentation. Instructions can comprise one or multiple documents, and are meant to include future updates.

"Subject" as used herein, means a human or a non-human mammal, e.g., a dog, a cat, a mouse, a rat, a cow, a sheep, a pig, a goat, a non-human primate or a bird, e.g., a chicken, as well as any other vertebrate or invertebrate.

The term "mammal" is used in its usual biological sense. Thus, it specifically includes, but is not limited to, primates, including simians (chimpanzees, apes, monkeys) and humans, cattle, horses, sheep, goats, swine, rabbits, dogs, cats, rodents, rats, mice, guinea pigs, or the like.

As used herein, "bacterial infection" refers to an infection caused by a species or strain of bacteria for which the uses disclosed herein are appropriate. The infection may be in any tissue or tissues of the subject. For example, the uses provided herein may be in the treatment of subjects having blood stream infections (BSI), catheter-related blood stream infections (CRBSI), osteomyelitis, prosthetic joint infections, pneumonia, joint space infections and device infections. The uses provided herein may be in the treatment of subjects having complicated skin and skin structure infections (cSSSI) and complicated and uncomplicated skin and soft tissue infections (SSTI), including abscesses, ulcers, burns, cellulitis, deep bacterial infections, such as major abscess, infected ulcer, major burn, or deep and extensive cellulitis. The uses for treatment can also be practiced concomitantly with surgical intervention for the bacterial infection.

An "effective amount" or a "therapeutically effective amount" as used herein refers to an amount of a therapeutic agent that is effective to relieve, to some extent, or to reduce the likelihood of onset of, one or more of the symptoms of a disease or condition, and includes curing a disease or condition. "Curing" means that the symptoms of a disease or condition are eliminated; however, certain long-term or permanent effects may exist even after a cure is obtained (such as extensive tissue damage).

"Treat," "treatment," or "treating," as used herein refers to administering a compound or pharmaceutical composition to a subject for prophylactic and/or therapeutic purposes. The term "prophylactic treatment" refers to treating a subject who does not yet exhibit symptoms of a disease or condition, but who is susceptible to, or otherwise at risk of, a particular disease or condition, whereby the treatment reduces the likelihood that the patient will develop the disease or condition. The term "therapeutic treatment" refers to administering treatment to a subject already suffering from a disease or condition.

### Methods of Preparation

As provided herein, the modified β-cyclodextrin may be from any source. As provided herein, the modified β-cyclodextrin may arise from enzymatic action, either in isolation or by the action of whole microbes, or may be synthesized by small molecule laboratory techniques, or any combination thereof. Methods of producing β-cyclodextrins may be found, for example, in U.S. Patent No. 4,384,898. The β-cyclodextrin may be purified, or may be provided in the presence of an insubstantial amount of contaminating materials. Purification of β-cyclodextrins may be accomplished by any suitable method, for example, those provided in U.S. Patent No. U.S. 4,808,232.

Means for the preparation of the glycopeptide antibiotics, including oritavancin and analogs thereof, may be found, for example, in U.S. Patent No. 5,840,684.

### Administration and Pharmaceutical Compositions

The compositions disclosed herein or the pharmaceutically acceptable salts thereof are administered intravenously.

The compositions disclosed herein are included in pharmaceutical formulations suitable for intravenous administration. In particular a pharmaceutical composition of the invention comprises:
oritavancin, or a salt thereof, and hydroxypropyl β-cyclodextrin (HPCD);
wherein the HPCD and oritavancin, or salt thereof, are present in a mass ratio of about 0.25:1 to about 4:1, wherein said composition is
   a) in solid form which on reconstitution provides an intravenous pharmaceutical composition; or
   b) in a solution, which is optionally diluted, which provides an intravenous pharmaceutical composition.

Standard pharmaceutical formulation techniques are used, such as those disclosed in Remington's The Science and Practice of Pharmacy, 21st Ed., Lippincott Williams & Wilkins (2005). Accordingly, some embodiments include pharmaceutical compositions comprising: (a) a safe and therapeutically effective amount of oritavancin, or pharmaceutically acceptable salts thereof; (b) a modified β-cyclodextrin; and (c) a pharmaceutically acceptable carrier, diluent, excipient or combination thereof.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Examples of classes of excipients generally used include, without limitation: stabilizing agents, solubilizing agents and surfactants, buffers, antioxidants and preservatives, tonicity agents, bulking agents, lubricating agents, emulsifiers, suspending or viscosity agents, inert diluents, fillers, disintegrating agents, binding agents, wetting agents, lubricating agents, antibacterials, chelating agents, sweeteners, perfuming agents, flavouring agents, coloring agents, administration aids, and combinations thereof. Except insofar as any conventional media or agent is incompatible with the active ingredient, its use in the therapeutic compositions is contemplated. In addition, various adjuvants such as are commonly used in the art may be included. Considerations for the inclusion of various components in pharmaceutical compositions are described, e.g., in Gilman et al. (Eds.) (1990); Goodman and Gilman's: The Pharmacological Basis of Therapeutics, 8th Ed., Pergamon Press.

Some examples of substances, which can serve as pharmaceutically-acceptable carriers or components thereof, are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions, to the extent that they are suitable for intravenous compositions.

The compositions described herein are preferably provided in unit dosage form. As used herein, a "unit dosage form" is a composition containing an amount of oritavancin that is suitable for administration to an animal, preferably mammal subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy. In a preferred embodiment, treatment includes a single administration. The skilled artisan will recognize that the formulation does not specifically contemplate the entire course of therapy and such decisions are left for those skilled in the art of treatment rather than formulation.

The compositions useful as described above is in a suitable form for intravenous administration. In light of the particular route of administration, a variety of pharmaceutically-acceptable carriers well-known in the art may be used. Pharmaceutically-acceptable carriers include, for example, liquid fillers, diluents, hydrotropies and surface-active agents. Optional pharmaceutically-active materials may be included, which do not substantially interfere with the biological activity. The amount of carrier employed is sufficient to provide a practical quantity of material for administration per unit dose. Techniques and compositions for making dosage forms useful in the methods described herein are described in the following references: Modern Pharmaceutics, 4th Ed., Chapters 9 and 10 (Banker & Rhodes, editors, 2002); Lieberman et al., Pharmaceutical Dosage Forms: Tablets (1989); and Ansel, Introduction to Pharmaceutical Dosage Forms 8th Edition (2004).

Compositions described herein may optionally include other drug actives.

For intravenous administration, the compositions described herein may be dissolved or dispersed in a pharmaceutically acceptable diluent, such as a saline or dextrose solution. A preferred diluent is 5% w/v dextrose in water ("D5W"). Suitable excipients may be included. Some excipients may be included to achieve the desired pH, including but not limited to NaOH, sodium carbonate, sodium acetate, HCl, and citric acid.

In various embodiments, the pH of the final composition ranges from about 1, about 1.5, about 2, about 2.5, about 3, about 3.5, about 4, about 4.5, about 5, about 5.5, about 6, about 6.5, about 7, about 7.5, or about 8 to about 1.5, about 2, about 2.5, about 3, about 3.5, about 4, about 4.5, about 5, about 5.5, about 6, about 6.5, about 7, about 7.5, about 8, about 8.5, about 9, or about 10. In some embodiments, the pH of the final composition ranges from about 2 to about 8, or from about 4.5 to about 8.

In some embodiments, antioxidant excipients may be included, such as sodium bisulfite, acetone sodium bisulfite, sodium formaldehyde, sulfoxylate, thiourea, and EDTA. In some embodiments, stabilizing agents may be included, such as carbohydrates, amino acids and polysorbates. In some embodiments, solubilizing agents may be included, such as cetrimide, sodium docusate, glyceryl monooleate, polyvinylpyrolidone (PVP) and polyethylene glycol (PEG). In some embodiments, surfactants may be included, such as polysorbates, tocopherol PEG succinate, poloxamer and Cremophor^{™}. In some embodiments, buffers may be included, such as acetates, citrates, phosphates, tartrates, lactates, succinates, amino acids and the like. In some embodiments, antioxidants or preservatives may be included, such as BHA, BHT, gentisic acids, vitamin E, ascorbic acid, sodium ascorbate and sulfur containing agents such as sulfites, bisulfites, metabisulfites, thioglycerols, thioglycolates and the like. In some embodiments, tonicity agents (for adjusting physiological compatibility), suspending or viscosity agents, chelating agents, and administration aids (e.g. local anesthetics, anti-inflammatory agents, anti-clotting agents, vasoconstrictors for prolongation and agents that increase tissue permeability) may be included. Antimicrobial agents may also be included to achieve a bacteriostatic or fungistatic solution, including but not limited to phenylmercuric nitrate, thimerosal, benzethonium chloride, benzalkonium chloride, phenol, cresol, and chlorobutanol. Other non-limiting examples of suitable excipients found in the final intravenous composition may include sodium or potassium phosphates, citric acid, tartaric acid, gelatin, and carbohydrates such as mannitol or dextran. Further acceptable excipients are described in Powell, et al., Compendium of Excipients for Parenteral Formulations, PDA J Pharm Sci and Tech 1998, 52 238-311 and Nema et al., Excipients and Their Role in Approved Injectable Products: Current Usage and Future Directions, PDA J Pharm Sci and Tech 2011, 65 287-332. In the materials and uses of the present disclosure, any combination of suitable excipients, including but not limited to those provided herein, may be included.

In some embodiments, oritavancin, or a salt thereof, and HPCD in a ratio as defined hereinbefore are present in an aqueous medium to provide an intravenous pharmaceutical composition. In certain embodiments, the aqueous medium is selected from the group consisting of water, normal saline, 5% dextrose in water, lactated ringer's solution or mixtures thereof.

In some embodiments, oritavancin, or a salt thereof, and HPCD in a ratio as defined hereinbefore are present in an aqueous medium that is free from undissolved material, for example an aqueous medium that is free from undissolved material following 24 hours of storage. Generally, storage can be conducted under any conditions typical for aqueous formulations. In various embodiments, storage can be carried out at room temperature, under typical refrigeration conditions as understood by persons in the art, which range from about 0 to 5 °C, under freezing conditions as understood by persons in the art, which range from about -5 to 0 °C, or under deep-freeze conditions as understood by persons in the art, which are below about -5 °C.

In some embodiments, carboxylic agents to increase solubility as described in U.S. Patent No. 5,646,131 may be included.

The compositions for intravenous administration may be provided to caregivers in the form of one more solids that are reconstituted with a suitable diluent such as water for injection ("WFI"), sterile water, saline or dextrose in water shortly prior to administration. In other embodiments, the compositions are provided in solution ready to administer parenterally. In still other embodiments, the compositions are provided in a solution that is further diluted prior to administration. In embodiments that include administering a combination of a compound described herein and another agent, the combination may be provided to caregivers as a mixture, or the caregivers may mix the two agents prior to administration, or the two agents may be administered separately. In some embodiments, the solid provided is a mixture that comprises, consists essentially, or consists of oritavancin, or a salt thereof, and the modified β-cyclodextrin. In some embodiments, the mixture of the oritavancin and modified β-cyclodextrin is a complex of the oritavancin with the modified β-cyclodextrin. In some embodiments, the mixture of the oritavancin and modified β-cyclodextrin is a mixture of oritavancin solids and modified β-cyclodextrin solids. In accordance with the invention, the modified β-cyclodextrin is 2-hydroxypropyl β-cyclodextrin, and the modified β-cyclodextrin and oritavancin, or salt thereof, are present in the mixture in a mass ratio of about 0.25:1 to about 4:1, for example, about 4:1.

To achieve intravenous administration, any suitable means for combining components of a treatment composition may be used. The compounds or compositions described herein may be dissolved or dispersed in a single composition, or in more than one composition. The compounds or compositions may be dissolved or dispersed in a single receptacle, such as an IV bag, or in more than one receptacle prior to administration. The components of the composition may be combined, for example, in a receptacle suitable for containing a treatment solution, in suitable means for transporting a treatment solution, such as a tube, or in the subject's bloodstream. The components of a treatment composition are combined to form a solution before administration. In some embodiments, a mixture of solids including oritavancin, or a salt thereof, and the modified β-cyclodextrin, can be dissolved in water to make an aqueous composition, for example, an aqueous solution. The concentration of oritavancin in the aqueous composition can be about 10 to 400 mg/mL, about 10 to 100 mg/mL, or about 20 to 75 mg/mL, for example, about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 150 mg/mL, about 200 mg/mL, about 300 mg/mL, or about 400 mg/mL. The aqueous composition can be further diluted with a suitable diluent to make a formulation of oritavancin, or a salt thereof, including the modified β-cyclodextrin ready for administration. The diluent may be selected from water, normal saline (for example, 0.9% saline), 5% dextrose in water, lactated ringer's solution, or mixtures thereof. The aqueous composition may be diluted with a selected amount of diluent such that the formulation has a final oritavancin concentration of about 1 to about 100 mg/mL, about 5 to 75 mg/mL, about 10 to about 50 mg/mL, or about 10 to about 20 mg/mL, for example, about 5 mg/mL, about 10 mg/mL, about 20 mg/mL, about 30 mg/mL, about 40 mg/mL, or about 50 mg/mL. The final concentration of the modified β-cyclodextrin generally will be determined by the desired ratio with oritavancin, or a salt thereof, and can be, for example, 10 mg/mL to 500 mg/mL. In some embodiments, solid oritavancin, or a salt thereof, can be diluted with an aqueous solution of the modified β-cyclodextrin, and the resulting aqueous composition of oritavancin, or a salt thereof, and the modified β-cyclodextrin can be further diluted, for example, with water, normal saline (for example, 0.9% saline), 5% dextrose in water, lactated ringer's solution, or mixtures thereof, to make a formulation that is ready to administer. In further embodiments, solid oritavancin, or a salt thereof, and optionally the modified β-cyclodextrin, may be distributed over a plurality of containers, for example vials, where the contents of each container can be dissolved with an aqueous medium, and the resulting solutions combined prior to, or during, administration.

In some embodiments, oritavancin, or a pharmaceutically acceptable salt, hydrate, solvate, or polymorph thereof, and the modified β-cyclodextrin, are provided as a ready-to-use solution in an aqueous medium. The aqueous medium may be selected from water, normal saline, 5% dextrose in water, lactated ringer's solution or mixtures thereof. In some embodiments, oritavancin, or a pharmaceutically acceptable salt, hydrate, solvate, or polymorph thereof, and the modified β-cyclodextrin, are provided as a solution in an aqueous medium, along with instructions for treating a bacterial infection.

### Lyophilization

In some embodiments, oritavancin and the modified β-cyclodextrin are present as a lyophilized powder. In some embodiments, a lyophilized powder may be produced by lyophilization of a formulation including oritavancin and the modified β-cyclodextrin. In some embodiments, a lyophilized powder is formed by lyophilizing a liquid formulation as provided herein. On reconstitution the lyophilized powder provides an intravenous pharmaceutical composition which is suitable for intravenous injection.

A "lyophilized powder" as provided herein includes all lyophilized formulations and constituents of formulations, including cakes, tablets, and the like. Lyophilization is a process whereby water is sublimed from a frozen liquid formulation. In this process, pharmaceutical and biological agents, and optionally excipients, can be dried without application of heat and stored in a dried state, often for extended periods of time. Persons of skill in the art may refer to available resources in preparing a lyophilized powder (see Remington's Pharmaceutical Sciences (18th ed., 1990)).

### Kits

Kits comprising oritavancin, or a pharmaceutically acceptable salt, hydrate, solvate, or polymorph thereof, the modified β-cyclodextrin, and optionally, instructions for treating a bacterial infection may be provided.

The oritavancin and the modified β-cyclodextrin may be coformulated or co-packaged. The oritavancin and the modified β-cyclodextrin may be provided as a lyophilized powder. The oritavancin and the modified β-cyclodextrin may be packaged with an additional active agent or agents. The kits may also comprise compounds and/or products co-packaged, coformulated, and/or co-delivered with other components. For example, a drug manufacturer, a drug reseller, a physician, a compounding shop, or a pharmacist can provide a kit comprising a disclosed compound and/or product and another component for delivery to a patient.

A ready-to-use kit can be used directly for delivery to a patient. One such kit includes a container and an aqueous solution of oritavancin and the modified β-cyclodextrin within the container. The aqueous solution can include oritavancin and modified β-cyclodextrin at concentrations that can be administered directly to a patient without further dilution. The container may be an intravenous bag. The intravenous bag may be stored in a frozen state prior to use and then thawed when it is to be used. T kit may include a plurality of containers, for example vials, each containing oritavancin, or a salt thereof, and the modified β-cyclodextrin, where the contents of the containers can be combined prior to administration.

In some embodiments, the concentration of oritavancin in a formulation for intravenous administration may be about 0.5 mg/mL, about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 70 mg/mL, about 80 mg/mL, about 90 mg/mL or about 100 mg/mL.

In some embodiments, the concentration of oritavancin in a formulation for intravenous administration may be from about 0.5 mg/mL, about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 70 mg/mL, about 80 mg/mL, or about 90 mg/mL to about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 70 mg/mL, about 80 mg/mL, about 90 mg/mL or about 100 mg/mL.

In some embodiments, the modified β-cyclodextrin and the oritavancin, or salt thereof, may be combined in a molar ratio of cyclodextrin to oritavancin of about 0.05, about 0.1, about 0.2, about 0.3, about 0.4, about 0.5, about 0.6, about 0.7, about 0.8, about 0.9, about 1, about 1.5, about 2, about 2.5, about 3, about 3.5, about 4, about 4.5, about 5, about 5.5, about 6, about 6.5, about 7, about 7.5, about 8, about 8.5, about 9, or about 10, providing a mass ratio of about 0.25:1 to about 4:1 is achieved.

In some embodiments, the modified β-cyclodextrin and the oritavancin, or salt thereof, may be combined in a molar ratio in a formulation from about 0.05, about 0.1, about 0.2, about 0.3, about 0.4, about 0.5, about 0.6, about 0.7, about 0.8, about 0.9, about 1, about 1.5, about 2, about 2.5, about 3, about 3.5, about 4, about 4.5, about 5, about 5.5, about 6, about 6.5, about 7, about 7.5, about 8, about 8.5, or about 9 to about 0.1, about 0.2, about 0.3, about 0.4, about 0.5, about 0.6, about 0.7, about 0.8, about 0.9, about 1, about 1.5, about 2, about 2.5, about 3, about 3.5, about 4, about 4.5, about 5, about 5.5, about 6, about 6.5, about 7, about 7.5, about 8, about 8.5, about 9 or about 10, providing a mass ratio of about 0.25:1 to about 4:1 is achieved.

In some embodiments, the volume of oritavancin in a formulation for intravenous administration may be about 10 mL, about 50 mL, about 100 mL, about 150 mL, about 160 mL, about 170 mL, about 180 mL, about 190 mL, about 200 mL, about 210 mL, about 220 mL, about 230 mL, about 240 mL, about 250 mL, about 160 mL, about 270 mL, about 280 mL, about 290 mL, about 300 mL, about 310 mL, about 320 mL, about 330 mL, about 340 mL, about 350 mL, about 400 mL, about 500 mL, about 600 mL, about 700 mL, about 800 mL or about 900 mL. In some embodiments, the volume of a formulation for intravenous administration including oritavancin may be selected so as to provide a single dose of oritavancin.

In some embodiments, the volume of a formulation for intravenous administration containing oritavancin may be from about 10 mL, about 50 mL, about 100 mL, about 150 mL, about 160 mL, about 170 mL, about 180 mL, about 190 mL, about 200 mL, about 210 mL, about 220 mL, about 230 mL, about 240 mL, about 250 mL, about 160 mL, about 270 mL, about 280 mL, about 290 mL, about 300 mL, about 310 mL, about 320 mL, about 330 mL, about 340 mL, about 350 mL, about 400 mL, about 500 mL, about 600 mL, about 700 mL, about 800 mL, about 900 mL, or about 1000 mL to about 50 mL, about 100 mL, about 150 mL, about 160 mL, about 170 mL, about 180 mL, about 190 mL, about 200 mL, about 210 mL, about 220 mL, about 230 mL, about 240 mL, about 250 mL, about 160 mL, about 270 mL, about 280 mL, about 290 mL, about 300 mL, about 310 mL, about 320 mL, about 330 mL, about 340 mL, about 350 mL, about 400 mL, about 500 mL, about 600 mL, about 700 mL, about 800 mL, about 900 mL or about 1000 mL.

In some embodiments, the time during which a formulation for intravenous administration containing oritavancin and the modified β-cyclodextrin is administered may be less than: about 0.1 hours, about 0.2 hours, about 0.3 hours, about 0.4 hours, about 0.5 hours, about 0.6 hours, about 0.7 hours, about 0.8 hours, about 0.9 hours, about 1 hour, about 1.25 hours, about 1.5 hours, about 1.75 hours, about 2 hours, about 2.25 hours, about 2.5 hours, about 2.75 hours, or about 3 hours.

In some embodiments, the time during which a formulation for intravenous administration containing oritavancin and the modified β-cyclodextrin is administered may be about 0.1 hours, about 0.2 hours, about 0.3 hours, about 0.4 hours, about 0.5 hours, about 0.6 hours, about 0.7 hours, about 0.8 hours, about 0.9 hours, about 1 hour, about 1.25 hours, about 1.5 hours, about 1.75 hours, about 2 hours, about 2.25 hours, about 2.5 hours, about 2.75 hours, or about 3 hours.

Hydrophobic carriers include, for example, lipids, for example, phospholids, and formulations may include polymer matrices, biocompatible polymers, lipospheres, vesicles, particles, and liposomes. Some formulations may include emulsions, for example, including emulsifiers such as phospholipids, poloxamers, polysorbates, and polyoxyethylene castor oil and osmotic agents such as sodium chloride, glycerol, sorbitol, xylitol and glucose. Liposomes include amphipathic lipids such as natural or derived phospholipids and optionally agents such as cholesterols or triacylglycerols.

Provided herein is a therapeutically effective amount of oritavancin. The therapeutically effective dosage will vary, for example, in view of the particular characteristics of the subject, concurrent medications administered to the subject, the severity of the subject's symptoms, the form of the infection, the identity of the bacteria, the location in the subject of the condition to be treated, the presentation of the condition in the subject, and the formulation and the means used to administer the drug formulation; the selection of the appropriate dose is well within the knowledge of the skilled artisan. The specific dose for a given subject is usually set by the judgment of the attending physician. A person of skill in the art may refer to available resources to determine a therapeutically effective dose, for example, U.S. Patent No. 8,420,592, and Orbactiv^{®} Prescribing Information.

In some embodiments, a daily dose may be from about 0.25 mg/kg to about 120 mg/kg or more of body weight, from about 0.5 mg/kg or less to about 50 mg/kg, from about 1.0 mg/kg to about 30 mg/kg of body weight, or from about 1.5 mg/kg to about 17 mg/kg of body weight. Thus, for administration to a 70 kg person, the dosage range would be from about 17 mg per day to about 8000 mg per day, from about 35 mg per day or less to about 3500 mg per day or more, from about 70 mg per day to about 2000 mg per day, or from about 100 mg per day to about 1200 mg per day.

In some embodiments, the therapeutically effective amount of oritavancin may be about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg oritavancin. In some embodiments, in each dose a therapeutically effective amount of oritavancin may be between about 100 mg and about 3000 mg oritavancin, or from about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg to about 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg. In a preferred embodiment, the therapeutically effective dose of oritavancin is about 1200 mg.

### Treatment of bacterial infection

Some embodiments of the present invention include treating a bacterial infection with the compositions described herein. Some methods include administering a composition described herein to a subject in need thereof. In some embodiments, a subject can be an animal, e.g., a mammal, a human.

In particular the invention provides a pharmaceutical composition comprising oritavancin, or a salt thereof, and hydroxypropyl β-cyclodextrin (HPCD) for use in a method of treating a bacterial infection comprising:
administering to a subject in need thereof a therapeutically effective amount of a pharmaceutical composition comprising oritavancin, or a salt thereof, HPCD, and an aqueous medium;
wherein the administration is intravenous;
wherein the HPCD and the oritavancin, or salt thereof, are present in the composition in a mass ratio of about 0.25: 1 to about 4:1.

Examples of bacterial infections include *Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Streptococcus intermedius, Streptococcus intermedius, Streptococcus constellatus, Streptococcus dysgalactiae, Streptococcus dysgalactiae subsp. equisimilis, Enterococcus faecalis, Enterococcus faecium,* vancomycin-resistant *Enterococcus faecalis, Enterococcus faecium,* vancomycin-resistant *Enterococcus faecium, Staphylococcus aureus,* methicillin-resistant *Staphylococcus aureus,* vancomycin-resistant *Staphylococcus aureus,* vancomycin-intermediate *Staphylococcus aureus,* vancomycin hetero-intermediate *Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis,* or *Staphylococcus saccharolyticus,* and combinations thereof. As provided herein, the bacterial infection treated may be caused by bacteria of any phenotype or genotype provided herein, or any other bacteria capable of causing a bacterial infection as provided herein. Bacteria may evolve over time, and any form of infective bacteria is within the scope of the present disclosure. Additional treatable bacteria may be discovered, for example, by methods described in U.S. Patent No. 8,815,535, and any such bacteria are within the scope of the present disclosure. As provided herein the infection treated in a subject may be due to more than one bacterial species, or due to more than one phenotype or genotype of a single bacterial species.

The infection treated may be a complicated skin and skin structure infection, as described in U.S. Patent No. 8,420,592.

In some embodiments, the subject is a human.

The treatments of the present disclosure may also be based on achieving a particular pharmacokinetic profile for oritavancin in a subject (Bhavnani et al., Antimicrobial Agents Chemother. 50(3):994-1000 (2006)). For example, the treating a bacterial infection in a subject in need thereof, comprising administering one dose of oritavancin, or a pharmaceutically acceptable salt thereof, in a composition as described hereinbefore, may be sufficient to achieve one or more of: (1) a maximum plasma concentration (Cmax) of oritavancin of a selected level, or (2) a minimum area under the concentration curve (AUC 0-24 hr) of a selected level.

Thus provided herein is a pharmaceutical composition comprising oritavancin, or a pharmaceutically acceptable salt thereof, the modified β-cyclodextrin, at a ratio as described hereinbefore, and a pharmaceutically acceptable carrier or diluent, for use in a method of treating a bacterial infection comprising: intravenously administering to a subject in need thereof, in an amount sufficient to achieve a maximum plasma concentration (Cmax) of oritavancin of not less than a selected value, or within a specified range. In a variation of this embodiment, a second dose of the pharmaceutical composition may also be administered to the subject.

It will be understood that a therapeutically effective Cmax of oritavancin will vary based on the concentration of oritavancin in the formulation being administered to a subject, the means of administration, the duration of administration, the type of bacterial infection being treated and the identity of the bacteria in the infection, among other factors such as the physical characteristics of the subject. In some embodiments, Cmax following administration may be from about 1, 5, 10, 15, 20, 25, 30, 35, 40, 45, 50, 55, 60, 65, 70, 75, 80, 85, 90, 95 or 100 mg/L in the subject to about 50, 55, 60, 65, 70, 75, 80, 85, 90, 95, 100, 110, 120, 130, 140, 150, 160, 170, 180, 190, 200, 210, 220, 230, 240, 250, 260, 270, or 280 mg/L. In some embodiments, the Cmax following intravenous administration of a composition including oritavancin as provided herein is substantially the same as the Cmax following intravenous administration of oritavancin in the absence of the modified β-cyclodextrin.

Further provided is a pharmaceutical composition comprising oritavancin, or a pharmaceutically acceptable salt thereof, the modified β-cyclodextrin, at a ratio as described hereinbefore, and a pharmaceutically acceptable carrier or diluent and a pharmaceutically acceptable carrier or diluent, for use in a method of treating a bacterial infection comprising: intravenously administering to a subject in need thereof, in an amount sufficient to achieve an area under the blood plasma concentration curve of oritavancin of at least a selected value over 24 hours following administration (AUC 0-24 hr). In a variation of this embodiment, a second dose of the pharmaceutical composition may also be administered to the subject.

It will be understood that a therapeutically effective minimum AUC 0-24 hr of oritavancin will vary based on the concentration of oritavancin in the formulation being administered to a subject, the means of administration, the duration of administration, the type of bacterial infection being treated and the identity of the bacteria in the infection, among other factors such as the physical characteristics of the subject. However, under most circumstances a minimum AUC 0-24 hr of at least about 20 µg*h/mL should be achieved in a subject. Thus, the present invention includes achieving a minimum AUC 0-24 hr of from about 300, 400, 500, 600, 700, 800, 900, 1000, 1100, 1200, 1300, 1400, 1500, 1750, 2000, 2250, or 2500 mg*h/L in the subject to about 400, 500, 600, 700, 800, 900, 1000, 1100, 1200, 1300, 1400, 1500, 1750, 2000, 2250, 2500, or 2750 mg*h/L. In preferred aspects, the AUC 0-24 hr of oritavancin is at least about 300 mg*h/L. In other preferred aspects, the AUC 0-24 hr of oritavancin is at least about 400 mg*h/L, 500 mg*h/L, or 600 mg*h/L.

Further provided is a pharmaceutical composition comprising oritavancin, or a pharmaceutically acceptable salt thereof, the modified β-cyclodextrin, at a ratio as described hereinbefore, and a pharmaceutically acceptable carrier or diluent and a pharmaceutically acceptable carrier or diluent, for use in a method of treating a bacterial infection comprising: intravenously administering to a subject in need thereof, in an amount sufficient to achieve an area under the blood plasma concentration curve, measured from administration until no active agent/metabolite is detectable (AUC 0-inf), of oritavancin, of at least a selected value. In a variation of this embodiment, a second dose of the pharmaceutical composition may also be administered to the subject.

The present invention includes achieving a minimum AUC 0-inf of from about 500, 600, 700, 800, 900, 1000, 1100, 1200, 1300, 1400, 1500, 1750, 2000, 2250, 2500, 2750, 3000, 3250, 3500, 3750, 4000, 4250, 4500, 4750, or 5000 mg*h/L in the subject to about 600, 700, 800, 900, 1000, 1100, 1200, 1300, 1400, 1500, 1750, 2000, 2250, 2500, 2750, 3000, 3250, 3500, 3750, 4000, 4250, 4500, 4750, 5000, or 5250 mg*h/L. In preferred aspects, the AUC 0-inf of oritavancin is at least about 1000 mg*h/L. In other preferred aspects, the AUC 0-inf of oritavancin is at least about 1100 mg*h/L, 1200 mg*h/L, 1300 mg*h/L, 1400 mg*h/L, 1500 mg*h/L, or 1600 mg*h/L.

Further embodiments include administering a combination of active compounds to a subject in need thereof. A combination can include oritavancin (in a composition as described hereinbefore) in combination with an additional medicament.

Some embodiments include co-administering oritavancin (in a composition as described hereinbefore) with an additional medicament. By "co-administration," it is meant that the two or more agents may be found in the subject at the same time, regardless of when or how they are actually administered. In one embodiment, the agents are administered simultaneously. In one such embodiment, administration in combination is accomplished by combining the agents in a single dosage form. In another embodiment, the agents are administered sequentially. In one embodiment the agents are administered through the same route. In another embodiment, the agents are administered through different routes, such as one being administered orally and the oritavancin being administered i.v.

Examples of additional medicaments include an antibacterial agent, a β-lactamase inhibitor, antifungal agent, an antiviral agent, an anti-inflammatory agent and an anti-allergic agent.

Also disclosed is a method of providing prophylaxis for bacterial infections in a subject by administering a dose of oritavancin, or a pharmaceutically acceptable salt thereof, and the modified β-cyclodextrin. The term "prophylaxis" refers to a reduction in the likelihood that a bacterial infection will develop in a subject, such as bacterial infection that may occur during or following a surgery, a dental procedure or other invasive medical procedure.

In one aspect, a subject receiving administration of oritavancin, or a pharmaceutically acceptable salt thereof, and the modified β-cyclodextrin may experience reduced administration site irritation, i.e. when the administration is by injection the site of administration is the injection site. The reduction in injection site irritation may be relative to the irritation experienced following injection of a formulation of oritavancin in the absence of the modified β-cyclodextrin. In some embodiments, the reduction in injection site irritation of a formulation containing oritavancin and the modified β-cyclodextrin is less than about 90%, 80%, 70%, 50%, 40%, 30%, 20%, of 10% of the irritation experienced following injection of a formulation of oritavancin in the absence of the modified β-cyclodextrin. The level of injection site irritation may be measured using known patient pain scales such as a visual analog scale (VAS).

To further illustrate this invention, the following examples are included. The examples should not, of course, be construed as specifically limiting the invention. The scope of the invention is as defined by the appended claims. The reader will recognize that the skilled artisan, armed with the present disclosure, and skill in the art is able to prepare and use the invention without exhaustive examples.

### EXAMPLES

### Example 1: Testing of formulations of oritavancin and a hydroxy propyl β-cyclodextrin

The following materials were used: HPCD (2-hydroxypropyl β-cyclodextrin); Orbactiv 400 mg/vial (Oritavancin); Oritavancin API; Active Fraction: 79.2%; 1N NaOH; 0.9% saline; Hank's Balanced Salt Solution (HBSS).

Reconstituting Orbactiv 400 mg (Oritavancin) with 50% HPCD. 5.915 g of HPCD was dissolved in 11.83 mL of water to make a 50 wt% aqueous solution. 8 mL of the 50% HPCD solution was added to a 400 mg vial of Orbactiv for a target oritavancin concentration of 50 mg/mL. Oritavancin went into solution within 5 minutes forming a clear, colorless solution. The pH was measured and the solution was stored at room temperature, protected from light, and observed over 24 hours for signs of precipitation **(Table 1).**

**Table 1: Orbactiv 400 mg (Oritavancin) Reconstituted using 50 % w/v HPCD in water**

| HPCD solution (% w/v) | Concentration of oritavancin (mg/mL) | Total Volume (mL) | pH | Time of Observation (hr) | Observations |
|---|---|---|---|---|---|
| 50 | 50 | 8.0 | 3.94 | 0 | Clear, colorless solution |
| 50 | 50 | 8.0 | ND | 24 | Clear, colorless solution |

The 50 mg/mL solution of oritvancin was further diluted in 0.9% saline to 5 mg/mL. The pH was measured and the solution was stored at room temperature, protected from light, and observed over 24 hours for signs of precipitation **(Table 2).**

**Table 2: Orbactiv 400 mg (Oritavancin) Reconstituted using 50 % w/v aqueous HPCD then further diluted in 0.9% saline**

| HPCD (% w/v) | Concentration of oritavancin (mg/mL) | Total Volume (mL) | pH | Time of Observatio n (hr) | Observations |
|---|---|---|---|---|---|
| 5 | 5 | 60.0 | 4.14 | 0 | Clear, colorless Solution |
| | | | 4.14 | 0.08 | Clear, colorless Solution |
| | | | 4.14 | 0.5 | Clear, colorless Solution |
| | | | 4.14 | 1 | Clear, Colorless Solution |
| | | | 4.15 | 24 | Clear, Colorless Solution |
| | | | 4.26 | 720 | Clear, Colorless Solution |

A 50% HPCD aqueous solution was able to reconstitute 400 mg Oritavancin at 50 mg/mL in 5 minutes to form a clear, colorless solution; over 24 hours the solution did not show any sign of visible precipitation. When further diluted to 5 mg/mL in 0.9% saline, the solution remained clear and colorless without any precipitation for 24 hours. When the solution was rechecked one month later, the solution was clear and colorless while the pH increased slightly.

Reconstituting Orbactiv 400 mg (Oritavancin) in 20 % w/v HPCD aqueous solution. 3.095 g HPCD was dissolved in 15.474 mL water to make a 20 % w/v solution (pH = 6.05). 8 mL of the 20 % w/v HPCD solution was added to a 400 mg vial of Orbactiv for a target oritavancin concentration of 50 mg/mL. Oritavancin went into solution within 5 minutes forming a clear, colorless solution. The pH was measured and the solution was stored at room temperature, protected from light, and observed over 24 hours for signs of precipitation **(Table 3).**

**Table 3: Orbactiv 400 mg (Oritavancin) Reconstituted using 20 % w/v HPCD in water**

| HPCD (% w/v) | Concentration of oritavancin (mg/mL) | Total Volume (mL) | pH | Time of Observatio n (hr) | Observations |
|---|---|---|---|---|---|
| 20 | 50 | 8.0 | 3.85 | 0 | Clear, colorless solution |
| | | | 4.17 | 24 | Clear, colorless solution |

The 50 mg/mL solution of oritavancin was further diluted in 0.9% saline to 5 mg/mL. The pH was measured and the solution was stored at room temperature, protected from light, and observed over 24 hours for signs of precipitation **(Table 4).**

**Table 4: Orbactiv 400 mg (Oritavancin) Reconstituted using 20 % w/v aqueous HPCD then further diluted in 0.9% saline**

| HPCD (% w/v) | Concentration of oritavancin (mg/mL) | Total Volume (mL) | pH | Time of Observatio n (hr) | Observations |
|---|---|---|---|---|---|
| 2 | 5 | 60.0 | 4.17 | 0 | Clear, colorless solution |
| | | | 4.17 | 0.08 | Clear, colorless solution |
| | | | 4.17 | 0.5 | Clear, colorless solution |
| | | | 4.17 | 1 | Clear, colorless solution |
| | | | 4.17 | 24 | Clear, colorless solution |
| | | | 4.32 | 720 | Clear, colorless solution |

A 20 % w/v HPCD solution was able to reconstitute 400 mg Oritavancin at 50 mg/mL in 5 minutes to form a clear, colorless solution; over 24 hours the solution did not show any sign of visible precipitation. When further diluted to 5 mg/mL in 0.9% saline, the solution remained clear and colorless without any precipitation for 24 hours. When the solution was rechecked one month later, the solution was clear and colorless while the pH increased slightly.

Reconstituting Orbactiv 400 mg (Oritavancin) in 20 % w/v HPCD, diluting in saline then adjusting pH. The 50 mg/mL solution of oritavancin in 20 % w/v HPCD (described in **Table 3**) was further diluted in 0.9% saline to 6 mg/mL in a small glass vial. The pH was adjusted using 1N NaOH. NaOH was added to the oritavancin solution in 5 µL increments and vortexed. Visual observations and pH were recorded **(Table 5).**

**Table 5: Orbactiv 400 mg (Oritavancin) Reconstituted using 20 % w/v aqueous HPCD then further diluted in 0.9% saline followed by pH adjustment using 1N NaOH**

| Base | Concentration of oritavancin (mg/mL) | Total Volume of base added (µL) | pH | Observations |
|---|---|---|---|---|
| 1N NaOH | 6.00 | 0 | 4.10 | Clear, colorless solution |
| | 5.99 | 5 | 5.33 | Clear, colorless solution |
| | 5.97 | 10 | 6.05 | Clear, colorless solution |
| | 5.96 | 15 | 6.32 | Solution becoming slightly milky and opaque |
| | 5.94 | 20 | 6.51 | Milky and opaque solution |

A 20 % w/v HPCD solution was able to reconstitute 400 mg Oritavancin at 50 mg/mL in 5 minutes to form a clear, colorless solution. When further diluted to 6 mg/mL in 0.9% saline, the solution remained clear and colorless without any visible precipitation. Adjusting pH with 1N NaOH beyond 6 caused visible precipitation of oritavancin from solution.

Reconstituting Orbactiv 400 mg (Oritavancin) in 50 % w/v HPCD aqueous solution, adjusting pH then diluting in saline. 6.269 g of HPCD was dissolved in 12.538 mL of water to make a 50 % w/v solution (pH = 7.23). Seven (7) mL of the 50 % w/v solution was added to a 400 mg vial of Orbactiv for a target oritavancin concentration of 57.14 mg/mL. Oritavancin went into solution within 5 minutes forming a clear, colorless solution. A 2 mL aliquot of 57.14 mg/mL was removed from the vial and placed in a small glass vial. The pH was adjusted to 6.5 using 1N NaOH. NaOH was added to the oritavancin solution in 5 µL increments and vortexed. Visual observations and pH were recorded **(Table 6).**

**Table 6: Orbactiv 400 mg (Oritavancin) Reconstituted using 50 % w/v aqueous HPCD then pH adjusted 1N NaOH**

| Base | Concentration of oritavancin (mg/mL) | Total Volume of base added (µL) | pH | Observations |
|---|---|---|---|---|
| 1N NaOH | 57.14 | 0 | 4.08 | Clear, colorless solution |
| | 57.10 | 5 | 4.26 | Clear, colorless solution |
| | 57.06 | 10 | 4.56 | Clear, colorless solution |
| | 57.02 | 15 | 4.90 | Clear, colorless solution |
| | 56.98 | 20 | 5.19 | Clear, colorless solution |
| | 56.94 | 25 | 5.39 | Clear, colorless solution |
| | 56.90 | 30 | 5.65 | Clear, colorless solution. |
| | | | | Further diluted to form solution #1 |
| | 56.86 | 35 | 5.81 | Clear, colorless solution. |
| | | | | Further diluted to form solution #2 |
| | 56.82 | 40 | 6.10 | Clear, colorless solution. |
| | | | | Further diluted to form solution #3 |
| | 56.78 | 45 | 6.17 | Clear, colorless solution. |
| | | | | Further diluted to form solution #4 |
| | 56.74 | 50 | 6.33 | Clear, colorless solution. |
| | | | | Further diluted to form solution #5 |

Five different aliquots with different pH values were prepared per **Table 7.** Aliquots as indicated in **Table 6** were diluted with 0.9% saline to a concentration as indicated in **Table 7.** Each vial was vortexed and pH measured. The solutions were stored at room temperature, protected from light, and observed for any visible precipitation for up to one hour **(Table 7).**

**Table 7: Orbactiv 400 mg (Oritavancin) Reconstituted using 50 % w/v aqueous HPCD, titrated using 1N NaOH, then further diluted in 0.9% saline**

| Solution | Concentration of oritavancin (mg/mL) | pH | Time of Observation (hr) | Observations |
|---|---|---|---|---|
| Solution #1 | 4.98 | 6.15 | 0 | Clear, colorless solution |
| | 4.98 | 6.15 | 0.08 | Clear, colorless solution |
| | 4.98 | ND | 1 | Clear, colorless solution |
| Solution #2 | 4.98 | 6.17 | 0 | Clear, colorless solution |
| | 4.98 | 6.17 | 0.08 | Clear, colorless solution |
| | 4.98 | ND | 1 | Clear, colorless solution |
| Solution #3 | 4.97 | 6.25 | 0 | Clear, colorless solution |
| | 4.97 | 6.25 | 0.08 | Clear, colorless solution |
| | 4.97 | ND | 1 | Clear, colorless solution |
| Solution #4 | 4.97 | 6.35 | 0 | Clear, colorless solution |
| | 4.97 | 6.35 | 0.08 | Clear, colorless solution |
| | 4.97 | ND | 1 | Clear, colorless solution |
| Solution #5 | 4.97 | 6.44 | 0 | Clear, colorless solution |
| | 4.97 | 6.44 | 0.08 | Clear, colorless solution |
| | 4.97 | ND | 1 | Clear, colorless solution |

50 % w/v aqueous HPCD solution was able to reconstitute 400 mg Orbactiv to 57 mg/mL in 5 minutes to form a clear, colorless solution. This could be pH adjusted to 6.3 without any sign of precipitation over an hour. When further diluted to 5 mg/mL in 0.9% saline, no precipitation was observed for at least 1 hour.

Reconstituting Orbactiv 400 mg (Oritavancin) in 50 % w/v aqueous HPCD, adjusting pH then diluting in saline. 6.112 g of HPCD was dissolved in 12.224 mL of water to make a 50 % w/v solution (pH = 6.74). Seven (7) mL of the 50 % w/v HPCD solution was added to a 400 mg vial of Orbactiv for a target oritavancin concentration of 57.14 mg/mL. Oritavancin went into solution within 5 minutes forming a clear, colorless solution. A 3 mL aliquot of 57.14 mg/mL was removed from the vial and placed in a small glass vial. The pH was adjusted to 7 using 1N NaOH. NaOH was added to the oritavancin solution in 5 µL increments and vortexed. Visual observations and pH were recorded **(Table 8).**

**Table 8: Orbactiv 400 mg (Oritavancin) Reconstituted using 50 % w/v aqueous HPCD then pH adjusted using 1N NaOH**

| Base | Concentratio n of oritavancin (mg/mL) | Total Volume of base added (µL) | pH | Total volume (mL) | Observations |
|---|---|---|---|---|---|
| 1N NaOH | 57.14 | 0 | 4.05 | 3.000 | Clear, colorless solution. |
| | | | | | Further diluted to form Unadjusted solution |
| | 57.04 | 5 | 4.28 | 2.830 | Clear, colorless solution |
| | 56.94 | 10 | 4.44 | 2.835 | Clear, colorless solution |
| | 56.84 | 15 | 4.68 | 2.840 | Clear, colorless solution |
| | 56.74 | 20 | 4.93 | 2.845 | Clear, colorless solution |
| | 56.64 | 25 | 5.11 | 2.850 | Clear, colorless solution |
| | 56.54 | 30 | 5.23 | 2.855 | Clear, colorless solution |
| | 56.44 | 35 | 5.36 | 2.860 | Clear, colorless solution |
| | 56.35 | 40 | 5.48 | 2.865 | Clear, colorless solution. |
| | | | | | Further diluted to form solution #6 |
| | 56.24 | 45 | 5.77 | 2.695 | Clear, colorless solution |
| | 56.14 | 50 | 5.82 | 2.700 | Clear, colorless solution |
| | 56.03 | 55 | 5.85 | 2.705 | Clear, colorless solution |
| | 55.93 | 60 | 5.94 | 2.710 | Clear, colorless solution |
| | 55.83 | 65 | 6.05 | 2.715 | Clear, colorless solution. |
| | | | | | Further diluted to form solution #7 |
| | 55.72 | 70 | 6.15 | 2.545 | Clear, colorless solution |
| | 55.61 | 75 | 6.20 | 2.550 | Clear, colorless solution |
| | 55.50 | 80 | 6.23 | 2.555 | Clear, colorless solution |
| | 55.39 | 85 | 6.34 | 2.560 | Clear, colorless solution |
| | 55.28 | 90 | 6.32 | 2.565 | Clear, colorless solution |
| | 55.17 | 95 | 6.38 | 2.570 | Clear, colorless solution |
| | 55.07 | 100 | 6.46 | 2.575 | Clear, colorless solution. |
| | | | | | Further diluted to form solution #8 |
| | 54.95 | 105 | 6.57 | 2.405 | Clear, colorless solution |
| | 54.84 | 110 | 6.59 | 2.410 | Clear, colorless solution |
| | 54.73 | 115 | 6.71 | 2.415 | Clear, colorless solution |
| | 54.61 | 120 | 6.70 | 2.420 | Clear, colorless solution |
| | 54.50 | 125 | 6.75 | 2.425 | Clear, colorless solution. |
| | | | | | Further diluted to form solution #9 |
| | 54.38 | 130 | 6.90 | 2.255 | Clear, colorless solution |
| | 54.26 | 135 | 6.90 | 2.260 | Clear, colorless solution |
| | 54.14 | 140 | 6.96 | 2.265 | Clear, colorless solution. |
| | | | | | Further diluted to form solution #10 |

As seen in **Table 9,** five solutions in saline were prepared starting with various aliquots as indicated in **Table 8,** after the pH had been adjusted to 5.5, 6, 6.5, 6.75, and 7. Each vial was vortexed and pH measured. The solutions were stored at room temperature, protected from light, and observed for any visible precipitation for 24 hours **(Table 9).**

**Table 9: Orbactiv 400 mg (Oritavancin) Reconstituted using 50 % w/v aqueous HPCD titrated using 1N NaOH then further diluted in 0.9% saline**

| Solution | Concentration of oritavancin (mg/mL) | HPCD (% w/v) | pH | Time of Observation (hr) | Observations |
|---|---|---|---|---|---|
| Unadjusted | 5 | 4.38 | 4.28 | 0 | Clear, colorless solution |
| | | | 4.71 | 12 | Clear, colorless solution |
| | | | 4.77 | 24 | Clear, colorless solution |
| Solution #6 | 4.93 | 4.32 | 5.92 | 0 | Clear, colorless solution |
| | | | 6.00 | 12 | Clear, colorless solution |
| | | | 5.93 | 24 | Visible particulates, slightly opaque |
| Solution #7 | 4.89 | 4.28 | 6.21 | 0 | Clear, colorless solution |
| | | | 6.27 | 12 | Clear, colorless solution |
| | | | 6.23 | 24 | Very few visible particulates, slightly opaque |
| Solution #8 | 4.82 | 4.22 | 6.60 | 0 | Clear, colorless solution |
| | | | 6.58 | 12 | Clear, colorless solution |
| | | | 6.58 | 24 | Slight change of color becoming slightly opaque |
| Solution #9 | 4.77 | 4.17 | 6.84 | 0 | Clear, colorless solution |
| | | | 6.85 | 12 | Clear, colorless solution |
| | | | 6.82 | 24 | Becoming slightly opaque |
| Solution #10 | 4.74 | 4.15 | 7.03 | 0 | Clear, colorless solution |
| | | | 7.11 | 12 | Clear, colorless solution |
| | | | 7.08 | 24 | Becoming very slightly opaque |

A 50 % w/v aqueous HPCD solution was able to reconstitute 400 mg Orbactiv to 57 mg/mL in 5 minutes to form a clear, colorless solution. This could be pH adjusted to 7 without any sign of precipitation over 24 hours. When further diluted to 5 mg/mL in 0.9% saline, no precipitation was observed for at least 12 hours. Some discoloration in solution was observed at 24 hours.

Reconstituting Orbactiv 400 mg (Oritavancin) in 20 % w/v aqueous HPCD, adjusting pH then diluting in saline. 3.555 g of HPCD was dissolved in 17.775 mL of water to make a 20 % w/v solution (pH = 6.88). Seven (7) mL of the 20 % w/v HPCD solution was added to a 400 mg vial of Orbactiv for a target oritavancin concentration of 57.14 mg/mL. Oritavancin went into solution within 5 minutes forming a clear, colorless solution. A 3 mL aliquot of 57.14 mg/mL was removed from the vial and placed in a small glass vial. The pH was adjusted to 7 using 1N NaOH. NaOH was added to the oritavancin solution in 5 µL increments and vortexed. Visual observations and pH were recorded. **(Table 10).**

**Table 10: Orbactiv 400 mg (Oritavancin) Reconstituted using 20 % w/v aqueous HPCD then pH adjusted 1N NaOH**

| Base | Concentration of oritavancin (mg/mL) | Total Volume of base added (µL) | pH | Total volume (mL) | Observations |
|---|---|---|---|---|---|
| | 57.14 | 0 | 3.95 | 3.000 | Clear, colorless solution. Further diluted to form Unadjusted solution |
| | 57.04 | 5 | 4.11 | 2.830 | Clear, colorless solution |
| | 56.94 | 10 | 4.31 | 2.835 | Clear, colorless solution |
| | 56.84 | 15 | 4.68 | 2.840 | Clear, colorless solution |
| | 56.74 | 20 | 4.89 | 2.845 | Clear, colorless solution |
| | 56.64 | 25 | 5.19 | 2.850 | Clear, colorless solution |
| | 56.54 | 30 | 5.29 | 2.855 | Clear, colorless solution |
| | 56.44 | 35 | 5.45 | 2.860 | Clear, colorless solution. |
| | | | | | Further diluted to form solution A |
| | 56.34 | 40 | 5.57 | 2.690 | Clear, colorless solution |
| | 56.23 | 45 | 5.63 | 2.695 | Clear, colorless solution |
| | 56.13 | 50 | 5.69 | 2.700 | Clear, colorless solution |
| | 56.03 | 55 | 5.82 | 2.705 | Clear, colorless solution |
| 1N NaOH | 55.92 | 60 | 5.87 | 2.710 | Clear, colorless solution |
| | 55.82 | 65 | 5.95 | 2.715 | Clear, colorless solution. |
| | | | | | Further diluted to form solution B |
| | 55.71 | 70 | 6.01 | 2.545 | Clear, colorless solution |
| | 56.60 | 75 | 6.06 | 2.550 | Clear, colorless solution |
| | 55.49 | 80 | 6.10 | 2.555 | Clear, colorless solution |
| | 55.38 | 85 | 6.18 | 2.560 | Clear, colorless solution |
| | 55.28 | 90 | 6.20 | 2.565 | Clear, colorless solution |
| | 55.17 | 95 | 6.24 | 2.570 | Clear, colorless solution |
| | 55.06 | 100 | 6.28 | 2.575 | Clear, colorless solution |
| | 54.95 | 105 | 6.34 | 2.580 | Clear, colorless solution |
| | 54.85 | 110 | 6.38 | 2.585 | Clear, colorless solution |
| | 54.74 | 115 | 6.44 | 2.590 | Clear, colorless solution |
| | 54.64 | 120 | 6.49 | 2.595 | Clear, colorless solution. |
| | | | | | Further diluted to form solution C |
| | 54.52 | 125 | 6.52 | 2.425 | Clear, colorless solution |
| | 54.41 | 130 | 6.54 | 2.430 | Clear, colorless solution |
| | 54.30 | 135 | 6.57 | 2.435 | Clear, colorless solution |
| | 54.19 | 140 | 6.62 | 2.440 | Clear, colorless solution |
| | 54.09 | 145 | 6.65 | 2.445 | Clear, colorless solution |
| | 53.97 | 150 | 6.69 | 2.450 | Clear, colorless solution |
| | 53.86 | 155 | 6.72 | 2.455 | Clear, colorless solution |
| | 53.75 | 160 | 6.76 | 2.460 | Clear, colorless solution. |
| | | | | | Further diluted to form solution D |
| | 53.63 | 165 | 6.80 | 2.290 | Clear, colorless solution |
| | 53.51 | 170 | 6.82 | 2.295 | Clear, colorless solution |
| | 53.40 | 175 | 6.86 | 2.300 | Clear, colorless solution |
| | 53.28 | 180 | 6.90 | 2.305 | Clear, colorless solution |
| | 53.17 | 185 | 6.93 | 2.310 | Clear, colorless solution |
| | 53.05 | 190 | 6.98 | 2.315 | Clear, colorless solution |
| | 52.94 | 195 | 7.03 | 2.320 | Clear, colorless solution. |
| | | | | | Further diluted to form solution E |

As seen in **Table 10,** five solutions in saline were prepared starting with various aliquots as indicated in **Table 9,** after the pH had been adjusted to 5.5, 6, 6.5, 6.75, and 7. Each vial was vortexed and pH measured. The solutions were stored at room temperature, protected from light, and observed for any visible precipitation for 24 hours **(Table 11).**

**Table 11: Orbactiv 400 mg (Oritavancin) Reconstituted using 20 % w/v aqueous HPCD titrated using 1N NaOH then further diluted in 0.9% saline**

| Solution | Concentration of oritavancin (mg/mL) | HPCD (% w/v) | pH | Time of Observation (hr) | Observations |
|---|---|---|---|---|---|
| Unadjusted | 5 | 1.75 | 4.29 | 0 | Clear, colorless solution |
| | | | 4.60 | 12 | Clear, colorless solution |
| | | | 4.72 | 24 | Clear, colorless solution |
| Solution A | 4.94 | 1.73 | 5.77 | 0 | Clear, colorless solution |
| | | | 5.78 | 12 | Clear, colorless solution |
| | | | 5.70 | 24 | Slight change of color becoming slightly opaque |
| Solution B | 4.88 | 1.71 | 6.06 | 0 | Clear, colorless solution |
| | | | 6.18 | 12 | Clear, colorless solution |
| | | | 6.17 | 24 | Slight change of color becoming slightly opaque |
| Solution C | 4.78 | 1.67 | 6.51 | 0 | Clear, colorless solution |
| | | | 6.62 | 12 | Clear, colorless solution |
| | | | 6.61 | 24 | Clear, colorless solution |
| Solution D | 4.70 | 1.65 | 6.80 | 0 | Clear, colorless solution |
| | | | 6.88 | 12 | Clear, colorless solution |
| | | | 6.85 | 24 | Clear, colorless solution |
| Solution E | 4.63 | 1.62 | 7.05 | 0 | Clear, colorless solution |
| | | | 7.18 | 12 | Clear, colorless solution |
| | | | 7.15 | 24 | Clear, colorless solution |

20 % w/v aqueous HPCD solution was able to reconstitute 400 mg Orbactiv to 57 mg/mL in 5 minutes to form a clear, colorless solution. This could be pH adjusted to 7 without any sign of precipitation over 24 hours. When further diluted to 5 mg/mL in 0.9% saline, no precipitation was observed for at least 12 hours. Some discoloration in solution was observed at 24 hours at pH 5.7-6.18. At pH 6.5 and above, solution maintained clear and colorless for 24 hours.

Reconstituting Orbactiv 400 mg (Oritavancin) in 20 % w/v aqueous HPCD then diluting in Hank's Balanced Salt Solution (HBSS). 2.641 g of HPCD was dissolved in 13.205 mL of water to make a 20 % w/v solution (pH = 6.70). Eight (8) mL of the 20 % w/v solution was added to a 400 mg vial of Orbactiv for a target oritavancin concentration of 50 mg/mL. Oritavancin went into solution within 5 minutes forming a clear, colorless solution. A 1.5 mL aliquot of 50 mg/mL was removed from the vial and placed in a small glass vial and pH measured. Diluted 2 mL of 50 mg/mL oritavancin solution in 8 mL of HBSS. Visual observations and pH were recorded. **(Table 12).**

**Table 12: Orbactiv 400 mg (Oritavancin) Reconstituted using 20 % w/v aqueous HPCD then further diluted to 10 mg/mL in HBSS**

| Concentration of oritavancin (mg/mL) | HPCD (% w/v) | pH | Time of Observation (hr) | Observations |
|---|---|---|---|---|
| 10 | 4 | 5.83 | 0 | Clear, colorless solution |
| | | 5.86 | 0.5 | Clear, colorless solution |
| | | 5.87 | 1 | Clear, colorless solution |
| | | 5.92 | 2 | Flocculent white precipitate |

HBSS was used as a plasma surrogate in this experiment. Oritavancin at either 5 or 10 mg/mL precipitated from solution at 2 hours in HBSS. This experiment was repeated with the same material 24 hours later and observed over shorter time intervals. Diluted 0.5 mL of 50 mg/mL oritavancin in 20 % w/v aqueous HPCD into 4.5 mL HBSS in a small glass vial to make a 10 mg/mL solution. Diluted 0.25 mL of 50 mg/mL oritavancin in 20 % w/v aqueous HPCD into 4.75 mL HBSS in a small glass vial to make a 5 mg/mL solution. Solutions were observed and pH was tested every 0.25 hrs. **(Table 14, Table 15).**

**Table 14: Orbactiv 400 mg (Oritavancin) Reconstituted using 20 % w/v aqueous HPCD then further diluted to 10 mg/mL in HBSS**

| Concentration of oritavancin (mg/mL) | HPCD (% w/v) | pH | Time of Observation (hr) | Observations |
|---|---|---|---|---|
| 10 | 2 | 6.19 | 0 | Clear, colorless solution |
| | | 6.21 | 0.25 | Clear, colorless solution |
| | | 6.30 | 0.5 | Very faint particulates, colorless solution |
| | | 6.33 | 0.75 | Faint particulates, colorless solution |
| | | 6.38 | 1 | Faint particulates, slightly opaque solution |

**Table 15: Orbactiv 400 mg (Oritavancin) Reconstituted using 20 % w/v aqueous HPCD then further diluted to 5 mg/mL in HBSS**

| Concentration of oritavancin (mg/mL) | HPCD (% w/v) | pH | Time of Observation (hr) | Observations |
|---|---|---|---|---|
| 5 | 2 | 6.39 | 0 | Clear, colorless solution |
| | | 6.47 | 0.25 | Clear, colorless solution |
| | | 6.50 | 0.5 | Clear, colorless solution |
| | | 6.59 | 0.75 | Clear, colorless solution |
| | | 6.61 | 1 | Clear, colorless solution |
| | | 6.63 | 1.25 | Very faint and miniscule |
| | | | | particulates starting to form, colorless solution |
| | | 6.71 | 1.5 | Faint particulates, colorless solution |
| | | 6.76 | 1.75 | Faint particulates, colorless solution |

Using HBSS as a plasma surrogate, 10 mg/mL oritavancin started to fall out of solution after 0.5 hrs. Five (5) mg/mL oritavancin started to fall out of solution at ~1.5 hrs. pH difference was observed with solution between time of reconstitution and solution used 24 hours later. An additional study will be performed using fresh material reconstituted in 20% HPCD and diluted to 10 and 5 mg/mL in HBSS. pH will be measured and solution observed every 0.25 hrs.

These several experiments show that oritavancin can be reconstituted using aqueous solutions of 2-hydroxypropyl β-cyclodextrin at both 20 % w/v and 50 % w/v. This solution can be diluted into saline at 5 mg/mL, and does not fall out of solution for at least one month. pH adjustment of solution to pH 7 in saline using NaOH showed that solution can maintain clarity for at least 12 hours. When oritavancin in 20 % w/v aqueous HPCD is diluted into HBSS at 5 mg/mL it will remain in solution for at least 1 hour.

### Example 2: Double blind study of pharmacokinetics of new formulations

A randomized, double-blind, single center cohort study of a new formulation of a single 1200mg IV dose of oritavancin in healthy volunteers, adjusting infusion time, concentration and reconstitution/administration solutions, of a single 1200 mg intravenous (IV) infusion of oritavancin in healthy adult subjects, was performed. Cohort 1 was administered the current, approved formulation of oritavancin which uses Sterile Water For Injection (SWFI) as the reconstituting agent and D5W for further dilution for a volume of 1000 mL. This infusion was given per the approved label over three hours. Cohort 2 was administered a new formulation for which hydroxypropyl-β-cyclodextrin (HPBCD) was included in the reconstitution diluent and D5W is used for further dilution for a total volume of 250 mL. This formulation was administered over two hours. Cohorts 3, 4, and 5 were administered the same new formulation as Cohort 2 (HPBCD and 250 mL of D5W); however, the infusion was administered over 90, 60, 30 minutes respectively. Two subjects in each of Cohorts 2, 3, 4, and 5 received a placebo infusion of D5W to use as a comparison when reviewing adverse event data. Healthy volunteer subjects providing informed consent and meeting all study eligibility criteria were randomized in the study and received one 1200 mg dose of IV oritavancin or placebo (cohorts 2-5 only).

Forty-six healthy subjects were enrolled in the study (approximately 50% men and 50% women) between the ages of 18 and 65 years.

For each cohort, oritavancin was supplied as a lyophilized powder in vials. Each vial contained 400 mg of oritavancin. Three 400 mg vials were required for each subject receiving oritavancin.

COHORT 1: At the time of use, each vial was reconstituted by adding 40 mL of Sterile Water or for Injection, ("SWFI," United States Pharmacopeia) to each 400 mg vial of oritavancin. Vials reconstituted in this manner gave a 10 mg/mL solution. After reconstitution, the compositions were combined and further diluted in 5% dextrose in water (D5W) to provide a total volume of approximately 1000 mL. At each dosing, 1200 mg of oritavancin in 1000 mL total of diluent was administered at a constant rate IV infusion over 3 hours via a single dedicated peripheral IV line (n=6).

COHORT 2: At the time of use, each vial was reconstituted by adding 4 mL of SWFI and 4 mL of 20% (w/v) HPBCD solution, to each 400 mg vial of oritavancin. Vials reconstituted in this manner provided a 50 mg/mL oritavancin solution. After reconstitution, the compositions were combined and further diluted in 5% dextrose in water (D5W) to provide a total volume of approximately 250 mL. At each dosing, 1200 mg of oritavancin in 250 mL of diluent was administered as a constant rate IV infusion over 2 hours via a single dedicated peripheral IV line (n=8) or a 2 hour placebo IV infusion of 250 mL of D5W (n=2).

COHORT 3: At the time of use, each vial was reconstituted by adding 4 mL of SWFI and 4 mL of 20% (w/v) HPBCD solution, to each 400 mg vial of oritavancin. Vials reconstituted in this manner provided a 50 mg/mL oritavancin solution. After reconstitution, the compositions were combined and further diluted in 5% dextrose in water (D5W) to provide a total volume of approximately 250 mL. At each dosing, 1200 mg of oritavancin in 250 mL of diluent was administered as a constant rate IV infusion over 90 minutes via a single dedicated peripheral IV line (n=8) or a 90 minute placebo infusion of 250 mL of D5W (n=2).

COHORT 4: At the time of use, each vial was reconstituted by adding 4 mL of SWFI and 4 mL of 20% (w/v) HPBCD solution, to each 400 mg vial of oritavancin. Vials reconstituted in this manner provided a 50 mg/mL oritavancin solution. After reconstitution, the compositions were combined and further diluted in 5% dextrose in water (D5W) to provide a total volume of approximately 250 mL. At each dosing, 1200 mg of oritavancin in 250 mL of diluent was administered as a constant rate IV infusion over 60 minutes via a single dedicated peripheral IV line (n=8) or a 60 minute placebo infusion of 250 mL of D5W (n=2).

COHORT 5: At the time of use, each vial was reconstituted by adding 4 mL of SWFI and 4 mL of 20% (w/v) HPBCD solution, to each 400 mg vial of oritavancin. Vials reconstituted in this manner provided a 50 mg/mL oritavancin solution. After reconstitution, oritavancin was further diluted in 5% dextrose in water (D5W) to provide a total volume of approximately 250 mL. At each dosing, 1200 mg of oritavancin in 250 mL of diluent was administered as a constant rate IV infusion over 30 minutes via a single dedicated peripheral IV line. (n=8) OR a 30 minute placebo infusion of 250 mL of D5W (n=2).

COHORT 6: At the time of use, each vial was reconstituted by adding 4 mL of SWFI and 4 mL of 20% (w/v) HPBCD solution, to each 400 mg vial of oritavancin. Vials reconstituted in this manner provided a 50 mg/mL oritavancin solution. After reconstitution, oritavancin was further diluted in 0.9% sodium chloride injection, or Normal Saline (NS) to provide a total volume of approximately 250 mL. At each dosing, 1200 mg of oritavancin in 250 mL of diluent was administered as a constant rate IV infusion over 60 minutes via a single dedicated peripheral IV line (n=8), or a 60 minute placebo infusion of 250 mL of NS (n=2).

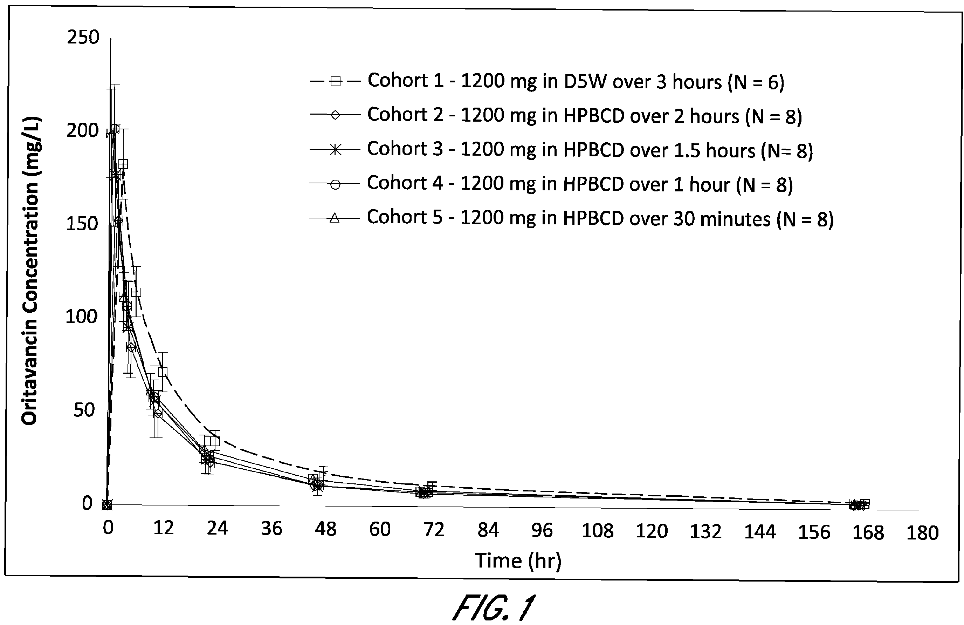

Prior to the start of dosing (T=0) and at the end of the infusion (either T=3 hours, T=2 hours, T=90 mins, T=60 mins or T=30 mins) the subjects had blood collected for the oritavancin plasma concentration analyses. The subjects had additional samples collected again 3 hours, 9 hours, 21 hours, 45 hours, 69 hours later and 165 hours after the completion of the infusion. The subjects underwent a final blood draw on Day 8 (168 Hours). The PK measurement results are provided in **Table 16 (****Figure 1****).** From the time of dosing until 7 days after dose administration, safety was evaluated by the assessment of adverse events (AEs)/serious adverse events (SAEs), clinical safety laboratory results, vital sign measurements and physical examination findings. Subjects returned on Day 15 (336 Hours) post dose administration to collect plasma for immunoglobulin levels and oritavancin antibodies, direct and indirect Coombs Test and evaluate safety by the assessment of adverse events, serious adverse events and record concomitant medications.

**Table 16: Average Cmax and AUC data for Cohort populations of Example 2; the "ORBACTIV^{®}" data was acquired following administration of oritavancin (as ORBACTIVO) to subjects in accordance with the Orbactiv^{®} Package Insert, as indicated in Comparative Example 4.**

| Parameter | Cohort 1 | Cohort 2 | Cohort 3 | Cohort 4 | Cohort 5 | Cohort 6 | Orbactiv |
|---|---|---|---|---|---|---|---|
| Infusion time | 3h | 2h | 1.5h | 1h | 0.5h | 1h | 3h |
| Diluent | D5W | D5W | D5W | D5W | D5W | NS | D5W |
| Cmax (mg/L) | 183 | 153 | 177 | 202 | 199 | 206 | 138 |
| AUC 0-inf (mg*h/L) | 3681 | 2713 | 2851 | 2867 | 3183 | 2962 | 2800 |

### Example 3: Study of pharmacokinetics of new formulations

Orbactiv 400 mg/vials were obtained from The Medicines Company.

Orbactiv in D5W: 20 mL of D5W was added to a 400 mg vial of Orbactiv (resulting in 20 mg/ml oritavancin). This solution was used as is for the high dose (100 mg/kg) testing and was further diluted to 10 mg/mL using D5W for the low dose (50 mg/kg) testing.

Orbactiv in HPβCD: 2.641 g of 2-hydroxypropyl β-cyclodextrin was dissolved in 13.2 mL of water to make a 20 % w/v aqueous solution (pH = 6.70). Eight mL of the 20 % w/v HPβCD solution was added to a 400 mg vial of Orbactiv for a target oritavancin concentration of 50 mg/mL oritavancin. This solution was diluted in 0.9% saline to 20 mg/mL for the high dose (100 mg/kg) and to 10 mg/mL for the low dose (50 mg/kg).

The study was conducted on Sprague-Dawley strain of rats, using male and female subjects from Charles River (Hollister, CA), with a body weight range of 200-230 g. Cannulation of the oritavancin formulation was via the femoral or jugular vein. For toxicology, ten subjects were given per dose & formulation, 5 male and 5 female, while six subjects per formulation, 6 male, for pharmacokinetics.

Upon receipt, the animals were individually housed in a room with a controlled environment and were acclimated to laboratory conditions for at least 24 hours prior to the start of dosing. Animals were provided food and water ad libitum. The health status of the animal was determined during the acclimation period. Each animal had its tail marked with indelible ink and each cage was identified by animal, group and study number.

Doses were calculated based on the body weight on the day of infusion.

Pharmacokinetic study - After acclimation, 6 male rats were marked, placed in individual cages and administered a single 50 or 100 mg/kg dose of oritavancin in D5W or HPβCD by intravenous infusion over 1 hour via an indwelling femoral vein cannula. Blood samples (~0.3 mL) were collected from each rat at designated timepoints up to 78 hours following the initiation of the infusion **(Table 17)** via an indwelling jugular vein cannula. Whole blood samples were placed in EDTA containing microcentrifuge tubes and immediately processed by the bioanalytical team. Whole blood was selected as for analysis in these studies due to experiments undertaken to determine the best recovery of expected concentrations from spiked samples. In serum and plasma, there was poor recovery of expected drug concentrations when spiked at concentrations greater than ~ 100 mg/L. In whole blood, the recovery was 100% up to concentrations of 500 mg/L (highest concentration tested).

**Table 17. Pharmacokinetic Study Design for Example 3**

| **Formulation** | **Dose Levels (mg/kg)** | **Whole Blood Sample Times (hr)** | **Tissue Sample Times (hr)** | **Dose Volume (mL/kg)*** | **Number of Animals per Formulation** |
|---|---|---|---|---|---|
| Orbactiv in D5W | 50 | 0.5, 1, 2, 4, 6, 8, 12, 20, 30, 48, 52, 70, 78 | 30, 52, 78 | 5 | 6 Males |
| Orbactiv in HPβCD | | | | | |
| Orbactiv in D5W | 100 | 0.5, 1, 2, 4, 6, 8, 12, 20, 30, 48, 52, 70, 78 | 30, 52, 78 | 5 | 6 Males |
| Orbactiv in HPβCD | | | | | |

Two rats from each formulation were sacrificed at 30, 52, and 78 hours after the start of infusion for tissue collections (thymus, lung, heart, liver, kidney, spleen, and bone marrow). The collected tissues were kept frozen for future bioanalysis.

Toxicity study - After acclimation, both male and female rats were administered a single 50 or 100 mg/kg dose of oritavancin in D5W or HPβCD by intravenous infusion over 1 hour via an indwelling femoral vein cannula. Eight days following the infusion, animals were euthanized using carbon dioxide.

Clinical hematology, chemistry and coagulation evaluations were performed on all animals on days 0 (pre-infusion) and 8 (post-infusion). Blood samples were either collected via femoral vein cannula or heart puncture following euthanasia. Blood samples for coagulation (citrate heparin tube) and serum chemistry (no additive tubes) were centrifuged within 5 minutes of the collection at 3000 g for 10 minutes and sent frozen to IDEXX laboratory (West Sacramento, CA) for analysis. Blood collected in EDTA tubes were sent without centrifugation for hematological evaluation.

Tissues including adrenals, thymus, heart, lung, spleen, liver, kidneys, and bone marrow (Tibia) were collected in neutral buffered 10% formalin jars for histology observations. All collected tissues were sent to IDEXX Laboratory (West Sacramento, CA) for embedding in paraffin wax, sectioning and staining with hematoxylin and eosin-phloxin. Blinded histological examination was performed by a board certified pathologist. All treatment and control groups are shown in **Table 18.**

**Table 18. Toxicology Study Design**

| **Group** | **Oritavancin Dose (mg/kg)** | **Oritavancin Concentratio n (mg/mL)** | **Dose Volume (mL/kg) *** | **Number of Animals** | |
|---|---|---|---|---|---|
| | | | | **Males** | **Female s** |
| HPβCD | 0 | 0 | 5 | 5 | 5 |
| Orbactiv in HPβCD | 50 | 10 | | | |
| Orbactiv in HPβCD | 100 | 20 | | | |
| 5% Dextrose in Water (D5W) | 0 | 0 | | 5 | 5 |
| Orbactiv in D5W | 50 | 10 | | | |
| Orbactiv in D5W | 100 | 20 | | | |

Whole Blood Analysis - Reference standards were prepared in D5W at a concentration of 10.0 mg/mL by reconstituting a 400 mg vial of Orbactiv in D5W. Reference standards were stored in a -80 °C freezer and thawed just prior to use. Calibration standards were prepared in duplicate in 0.5% formic acid in 50-50 methanol-water from dilutions of the 10.0 mg/mL reference standard stock solution at actual concentrations of: 1.00, 2.50, 5.00, 10.0, 25.0, 50.0, 100, 250 and 500 µg/mL for oritavancin. QCs were prepared in triplicate in Sprague-Dawley rat whole blood (Bioreclamations) on the same day from dilutions of the 10.0 mg/mL oritavancin reference standard stock solution at actual concentrations of 400, 100, 20.0 and 1.00 µg/mL.

The samples were prepared using a solid phase extraction procedure by combining 20.0 µL of each QC or sample in a 1.5 mL Eppendorf tube with 20.0 µL of 0.5% formic acid in 50-50 methanol-water, 20.0 µL of 50.0 µg/mL TT99000808 as the internal standard in 0.5% formic acid in 50-50 water-methanol and 500 µL of 1.0% formic acid in water. For the calibration standards, 20.0 µL of standard was combined in a 1.5 mL Eppendorf tube with 20.0 µL of blank Sprague-Dawley rat whole blood (Bioreclamations), 20.0 µL of 50.0 µg/mL TT99000808 as the internal standard in 0.5% formic acid in 50-50 water-methanol and 500 µL of 1.0% formic acid in water. A control standard made from blank whole blood and a matrix blank made from blank whole blood with no internal standard were included with each run as well. All samples, standards, QCs and blanks were vortexed then centrifuged for 5 min at 5000 rpm. Meanwhile Isolute C2 SPE columns (Biotage, 100 mg sorbent mass, 1mL reservoir volume) were conditioned by applying first 500 microliters of methanol followed by 500 µL of 1.0% formic acid in water and allowing each wash to elute completely.

Fifty microliters (50 µL) of the supernatant from each centrifuged sample mixture was transferred to a conditioned SPE column then washed with 500 µL of DI water followed by 500 µL of acetonitrile and 500 µL of 1:9 methanol:water v/v. The eluent from these washes was all sent to waste. Finally the analyte and internal standard were eluted using 200 µL of 1.0% formic acid in 60:30 methanol:water v/v followed by 200 µL of DI water. The eluted sample extracts were collected, vortexed then centrifuged for 5 min at 1000 G. The supernatant was transferred to a 96-well plate and stored at 5 degrees C pending injection on LC-MS. A calibration curve and three QCs at each concentration level were prepared with each run.

HPLC-MS Conditions for Whole Blood Analysis - Sixty microliters (60 µL) of sample extract for each sample was injected on an HPLC/MS/MS system consisting of an Agilent 1100 LC system, LEAP PAL autosampler with a 20.0 µL injection loop and AB Sciex 3200 QTrap mass spectrometer equipped with a Chromolith FastGradient RP-18e, 2x50 mm analytical column and Phenomenex Fusion RP guard column. Mobile phase A consisted of 0.5% formic acid in water and mobile phase B consisted of 0.5% formic acid in acetonitrile at a flow rate of 500 µL per minute with a starting ratio of 85% A and 15% B and a gradient to 45% B at 1.5 minutes, followed by a gradient to 80% B at 1.6 minutes. The quantitation of the analyte oritavancin was performed using peak area ratios. A 1/x^2 weighted linear regression was used to determine the concentrations of oritavancin. The data was acquired and integrated using Analyst 1.6. The mass transitions for analyte and IS were 598.615 > 100.1 and 596.258 > 195.1, respectively.

Data Analysis - Whole blood concentrations of oritavancin were fit using a compartmental model. These parameters were generated using Phoenix^{®}WinNonlin ^{®}Version 6.3 (Certara USA, Inc. Princeton).

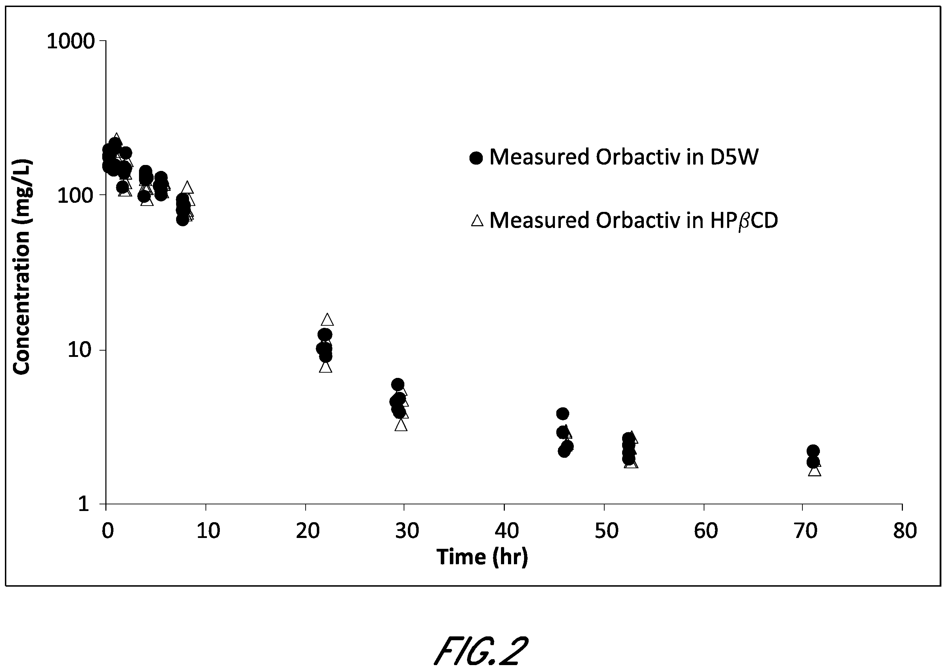

Pharmacokinetics: Whole Blood Pharmacokinetic Parameters of Orbactiv in D5W or HPβCD - As shown in **Figure 2** and **Table 19,** blood concentrations were similar for both formulations at each dose level. The maximum whole blood concentrations were achieved at the end of the 1 hour infusion for all rats ranging between 137-160 and 245-280 mg/L for 50 and 100 mg/kg, respectively. Infusion with 50 mg/kg of Orbactiv in D5W or HPβCD had AUCs of 1007.17 and 895.41 mg*hr/L in whole blood, respectively. Infusion with 100 mg/kg of Orbactiv in D5W or HPβCD had AUCs of 1992.15 and 1964.47 mg*hr/L in whole blood, respectively. Due to issues with drug recovery, as described above, whole blood was collected as opposed to plasma. In order to directly compare exposures achieved in this study with human plasma exposures, we used the whole blood vs plasma AUC relationship from radiolabeled PK study ADME-rpt-7-053r95. In this study, the whole blood AUC was 62.8% of the plasma AUC or, stated another way, the plasma AUC was 1.59X the whole blood AUC. Using this conversion, the plasma AUCs of rats infused with 50 mg/kg were 1603.77 and 1425.81 mg*hr/L in D5W and HPβCD, respectively. The mean plasma AUCs of rats infused with 100 mg/kg were 3172.21 and 3128.14 mg*hr/L in D5W and HPβCD, respectively Overall, there were no differences in the pharmacokinetic profile of Orbactiv in D5W or HPβCD.

Pharmacokinetics: Standard Curve and Concentrations - The whole blood standard curve for oritavancin was linear between 1 and 500 µg/mL. Whole blood AUC was 62.8% of the plasma AUC. Plasma AUC was thus determined by multiplying the whole blood AUC by 1.59. Data is provided in **Table 19 (****Figure 2****).**

**Table 19. Whole Blood Pharmacokinetic Parameters of Oritavancin formulated in D5W or in HPβCD following a 1 hour Intravenous Infusion of 50 or 100 mg/kg in Male Sprague-Dawley Rats.**

| Compounds | Samples | Dose (mg/kg) | Cl (L/hr/kg) | AUC_{(0-∞)} (hr*mg/kg) | Cmax (mg/L) |
|---|---|---|---|---|---|
| Orbactiv in D5W | Whole Blood | 50 | 0.05±0.00 | 1007.17±39.32 | 159.89±7.78 |
| | *Plasma ** | *50* | | *1603.77* | |
| Orbactiv in HPβCD | Whole Blood | 50 | 0.06±0.00 | 895.41±35.85 | 137.81±6.91 |
| | *Plasma ** | *50* | | *1425.81* | |
| Orbactiv in D5W | Whole Blood | 100 | 0.05±0.00 | 1992.15±116.4 4 | 280.25±15.4 2 |
| | *Plasma ** | *100* | | *3172.21* | |
| Orbactiv in HPβCD | Whole Blood | 100 | 0.05±0.01 | 1964.47±261.0 3 | 244.52±15.8 3 |
| | *Plasma** | *100* | | *3128.14* | |

Toxicity: Clinical Observations - There were no physical signs or symptoms of toxicity nor any mortalities during the course of study with 50 or 100 mg/kg of oritavancin formulated in D5W or in HPβCD.

Serum Chemistry - Aspartate aminotransferase (AST) and creatinine kinase (CK) enzymes were the only significantly elevated serum chemistry values for both treated and control animals with both formulations (P <0.05). These increased values were observed when comparing pre-treated and post treated data. These findings indicate that elevated values were not treatment related since they occurred in the control groups as well. Glucose levels were significantly higher in all groups (pre-treatment) compared to 8 days post treatment. These values are falsely elevated due to the preparation of the cannula with 50% dextrose and heparin following cannulation. There were no dose or formulation effects on all other measured serum chemistry values in either male or female rats.

Hematology - There was no evidence of dose or formulation effects on hematological parameters. Eosinophil values were significantly higher when treated and control groups were compared on day 8. Statistical analysis of these differences lacked a dose or formulation response and were interpreted to be likely due biological variation and not related to the formulation.

Coagulation - There were no dose or formulation related changes in Prothrombin Time (PT) and Activated Partial Thromboplastin Time (APTT). Differences in PT were considered to be incidental since they were observed in the control groups of both oritavancin formulations. APTT values were higher than normal in some treated and control animals possibly due to the use of heparin during blood collection and as part of cannula maintenance.

Histopathology - There were no treatment-related microscopic changes in any animals with either formulation of oritavancin at either dose. No treatment-related changes were noted in any animals. Some of the more common microscopic findings, which occurred across all groups in both males and females, included: congestion in the heart; congestion, and occasionally edema, in the lung; and congestion in the liver of males. These changes are terminal and consistent with euthanasia (although the pattern of congestion in the lung and liver are somewhat unusual). Remaining microscopic observations were either common findings in the rat (i.e. extramedullary hematopoiesis in the spleen) or sporadic changes not uncommon in this species.

The pharmacokinetics of oritavancin formulated in D5W or HPβCD were compared after a one hour intravenous infusion of 50 or 100 mg/kg in male Sprague-Dawley rats. Data indicate that the formulation does not have an impact on the pharmacokinetics of oritavancin. Rats infused with 50 mg/kg of Orbactiv in D5W or HPβCD had maximum concentrations (Cmax) of 160 and 138 mg/L, respectively. Rats infused with 100 mg/kg of Orbactiv in D5W or HPβCD had Cmaxs of 244 and 280 mg/L, respectively. The whole blood AUCs at 100 mg/kg were 1992.15 and 1964.47 mg*hr/L and the estimated plasma AUCs at 100 mg/kg were 3172.21 and 3128.14 mg*hr/L for orbactiv in D5W and HPβCD, respectively. The concentrations of oritavancin in both formulations increased proportionally with dose.

In the toxicity study, there were no clinical observations, hematology or clinical chemistry changes due to oritavancin formulated in D5W or in HPβCD over 8 days following single 1-hour intravenous infusion of 50 or 100 mg/kg. There were no treatment related macroscopic or microscopic changes in tissues collected at any dose with either formulation. Based on the lack of any significant findings with either formulation of oritavancin, the NOAEL for oritavancin administered in D5W or in a HPβCD solution was found to be ≥ 100 mg/kg following a single 1 hour infusion.

In the rat toxicology study, the dose of HPβCD was 400 mg/kg in both the control group and high dose oritavancin group and was associated with no adverse findings in either group. The 400 mg/kg dose of HPβCD in the rat (using body surface conversions described by the FDA Guidance for Industry - Safe starting dose in man, 2005) is equivalent to ~ 4000 mg dose of HPβCD in man. Although the rat study used a 4:1 ratio of HPβCD to oritavancin, only a 2:1 ratio is required for the formulation to be used in humans; thus the human dose HPβCD was only 2400 mg in the single dose study with oritavancin. This dose of HPβCD is lower than that currently employed in other FDA approved antiinfective products that (in contrast to oritavancin) are given as multiple doses over several days; see the table below for comparisons of the amounts of HPβCD used with other drugs.

The plasma AUC of oritavancin formulated in HPβCD in the rat following a 100 mg/kg dose was 3128.14 mg*h/L. This exposure level is, roughly, equivalent to a 1200 mg dose in man (~ 2800 mg*h/L).

### Comparative Example 4: Pharmacokinetic parameters for a formulation of oritavancin not including a cyclodextrin

Oritavancin (as ORBACTIV^{®}) was evaluated in double-blind, controlled ABSSSI clinical trials, which included 976 adult patients treated with a single 1200 mg intravenous dose of oritavancin in 1000 mL D5W. The formulation was prepared by adding 40 mL of sterile water for injection (WFI) to each of three 400 mg vials of ORBACTIV^{®} to provide a 10 mg/mL solution per vial. Each vial was gently swirled to avoid foaming and ensure that all ORBACTIV powder was completely reconstituted in solution. Each vial was inspected visually for particulate matter after reconstitution and should appear to be clear, colorless to pale yellow solution. The three solutions were combined and diluted to 1000 mL total volume with 5% dextrose in sterile water (D5W), to give a 1.2 mg/mL solution of oritavancin. The formulation was administered to each subject intravenously over 3 hours, and PK was measured **(Table 20).** The median age of patients treated with oritavancin was 45.6 years, ranging between 18 and 89 years of age with 8.8% ≥65 years of age. Patients treated with oritavancin were predominantly male (65.4%), 64.4% were Caucasian, 5.8% were African American, and 28.1% were Asian. Safety was evaluated for up to 60 days after dosing.

**Table 20: Mean Pharmacokinetic Parameters for ABSSSI Subjects Receiving a Single 1200 mg Dose (n = 297). Vss: Steady-state volume of distribution; Cmax: Maximum plasma concentration; AUC0-24: Area under the plasma concentration-time curve from time zero to 24 hours, AUC0-∞: Area under the plasma concentration time curve from time zero to infinity; T1/2,α: Half-life for the alpha phase, T1/2,β: Half-life for the beta phase; T1/2,γ: Half-life for the gamma phase; CV%: Percent Coefficient of variation.**

| **Parameter** | **Mean** | **(CV%)** |
|---|---|---|
| Vₛₛ(L) | 97.8 | (56.4%) |
| Cₘₐₓ (µg/mL) | 138 | (23.0%) |
| AUC₀₋₂₄ (µg•h/mL) | 1110 | (33.9%) |
| AUC₀₋₇₂ (µg•h/mL) | 1530 | (36.9) |
| AUC_{0-∞} (µg•h/mL) | 2800 | (28.6%) |
| T_{1/2},_{α} (h) | 2.29 | (49.8%) |
| T_{1/2,β} (h) | 13.4 | (10.5%) |
| T_{1/2,γ} (h) | 245 | (14.9%) |

## Claims

1. A pharmaceutical composition comprising:
oritavancin, or a salt thereof, and hydroxypropyl β-cyclodextrin (HPCD);
wherein the HPCD and oritavancin, or salt thereof, are present in a mass ratio of about 0.25:1 to about 4:1, wherein said composition is
a) in solid form which on reconstitution provides an intravenous pharmaceutical composition; or
b) in a solution, which is optionally diluted, which provides an intravenous pharmaceutical composition.

2. A pharmaceutical composition of claim 1, wherein the HPCD and the oritavancin, or a salt thereof, are in a 2:1 (w/w) ratio.

3. The pharmaceutical composition of claim 1 or 2, wherein said intravenous pharmaceutical composition is in an aqueous medium.

4. The pharmaceutical composition of claim 3, wherein the oritavancin, or salt thereof, is present in the aqueous medium at a concentration of about 1.2 mg/mL to about 60 mg/mL; or optionally
wherein the oritavancin, or salt thereof, is present in the aqueous medium at a concentration of about 2 mg/mL to about 8 mg/mL.

5. The pharmaceutical composition of claim 3, wherein the pH of the composition is about 4 to about 8; or optionally
wherein the pH of the composition is about 4 to about 6.

6. The pharmaceutical composition of claim 3, wherein the aqueous medium is selected from the group consisting of water, normal saline, 5% dextrose in water, lactated ringer's solution or mixtures thereof.

7. The pharmaceutical composition of claim 3, wherein the composition is a solution:
(a) free from undissolved material;
(b) free from undissolved material following 24 hours of storage;
(c) free from undissolved material following 72 hours of storage; or
(d) free from undissolved material following 1 month of storage.

8. The pharmaceutical composition of claim 3, wherein the HPCD is present in a concentration of about 0.2% to about 1% w/v and oritavancin, or salt thereof, is present in a concentration of about 0.5% w/v; or
wherein the HPCD is present in a concentration of about 0.4% to about 2% w/v and oritavancin, or salt thereof, is present in a concentration of about 1% w/v.

9. The pharmaceutical composition of claim 1, wherein the solid form is a lyophilized powder; and/or
wherein oritavancin is present as a free base.

10. A pharmaceutical composition comprising oritavancin, or a salt thereof, and hydroxypropyl β-cyclodextrin (HPCD) for use in a method of treating a bacterial infection comprising:
administering to a subject in need thereof a therapeutically effective amount of a pharmaceutical composition comprising oritavancin, or a salt thereof, HPCD, and an aqueous medium;
wherein the administration is intravenous;
wherein the HPCD and the oritavancin, or salt thereof, are present in the composition in a mass ratio of about 0.25: 1 to about 4:1.

11. The pharmaceutical composition for use according to claim 10, wherein the HPCD and the oritavancin, or a salt thereof, are in a 2:1 (w/w) ratio.

12. The pharmaceutical composition for use according to claim 10 or 11, wherein the administering is completed:
(a) in less than 3 hours;
(b) in less than 2 hours;
(c) in less than 1.5 hours;
(d) in less than 1 hour; or
(e) in about 1 hour.

13. The pharmaceutical composition for use according to claim 10 or 11, wherein the composition is configured to be administered in a single dose.

14. The pharmaceutical composition for use according to claim 10 or 11, wherein the composition has a volume of from about 100 mL to about 500 mL; or optionally
wherein the composition has a volume of about 250 mL.

15. The pharmaceutical composition for use according to claim 10 or 11, wherein the composition has oritavancin, or salt thereof, at a concentration from about 1.2 mg/mL to about 60 mg/mL; or optionally
wherein the composition has oritavancin, or salt thereof, at a concentration from about 2 mg/mL to about 8 mg/mL.

16. The pharmaceutical composition for use according to claim 10 or 11, wherein the bacterial infection is caused by a gram positive microorganism selected from the group consisting of *Staphylococcus aureus,* methicillin-resistant *Staphylococcus aureus,* methicillin-susceptible *Staphylococcus aureus,* vancomycin-resistant *Staphylococcus aureus,* vancomycin-intermediate *Staphylococcus aureus,* vancomycin hetero-intermediate *Staphylococcus aureus, Streptococcus pyogenes, Streptococcus agalactiae, Streptococcus anginosus, Streptococcus intermedius, Streptococcus constellatus, Streptococcus dysgalactiae, Streptococcus dysgalactiae* subsp. *equisimilis, Enterococcus faecalis,* vancomycin-resistant *Enterococcus faecalis, Enterococcus faecium,* and vancomycin-resistant *Enterococcus faecium.*

17. The pharmaceutical composition for use according to claim 10 or 11, wherein the Cmax of oritavancin in the subject following administration is about 100 to about 300 mg/L; and/or
wherein the AUC 0-inf of oritavancin in the subject following a single administration is about 1000 to about 4000 mg*h/L; and/or
wherein the subject experiences reduced injection site irritation.

## Patentansprüche

1. Pharmazeutische Zusammensetzung, umfassend:
Oritavancin oder ein Salz davon und Hydroxypropyl-β-Cyclodextrin (HPCD);
wobei das HPCD und Oritavancin oder ein Salz davon in einem Massenverhältnis von etwa 0,25:1 bis etwa 4:1 vorhanden sind, wobei die Zusammensetzung:
a) in fester Form vorliegt, die bei Rekonstitution eine intravenöse pharmazeutische Zusammensetzung bereitstellt; oder
b) b) in einer Lösung ist, die gegebenenfalls verdünnt ist, wodurch eine intravenöse pharmazeutische Zusammensetzung bereitgestellt wird.

2. Pharmazeutische Zusammensetzung nach Anspruch 1, wobei das HPCD und das Oritavancin oder ein Salz davon in einem Verhältnis von 2:1 (Gew.-%) vorliegen.

3. Pharmazeutische Zusammensetzung nach Anspruch 1 oder 2, wobei die intravenöse pharmazeutische Zusammensetzung in einem wässrigen Medium vorliegt.

4. Pharmazeutische Zusammensetzung nach Anspruch 3, wobei das Oritavancin oder ein Salz davon in dem wässrigen Medium in einer Konzentration von etwa 1,2 mg/ml bis etwa 60 mg/ml vorhanden ist; oder
wobei das Oritavancin oder ein Salz davon gegebenenfalls in dem wässrigen Medium in einer Konzentration von etwa 2 mg/ml bis etwa 8 mg/ml vorhanden ist.

5. Pharmazeutische Zusammensetzung nach Anspruch 3, wobei der pH-Wert der Zusammensetzung etwa 4 bis etwa 8 beträgt; oder
wobei der pH-Wert der Zusammensetzung gegebenenfalls etwa 4 bis etwa 6 beträgt.

6. Pharmazeutische Zusammensetzung nach Anspruch 3, wobei das wässrige Medium ausgewählt ist aus der Gruppe bestehend aus Wasser, normaler Kochsalzlösung, 5 % Dextrose in Wasser, Lactat-Ringer-Lösung oder Mischungen davon.

7. Pharmazeutische Zusammensetzung nach Anspruch 3, wobei die Zusammensetzung eine Lösung ist:
(a) frei von ungelöstem Material;
(b) frei von ungelöstem Material nach 24 Stunden Lagerung;
(c) frei von ungelöstem Material nach 72 Stunden Lagerung; oder
(d) frei von ungelöstem Material nach 1 Monat Lagerung.

8. Pharmazeutische Zusammensetzung nach Anspruch 3, wobei das HPCD in einer Konzentration von etwa 0,2 % bis etwa 1 % Gew./Vol. und Oritavancin oder ein Salz davon in einer Konzentration von etwa 0,5 % Gew./Vol. vorhanden ist; oder
wobei das HPCD in einer Konzentration von etwa 0,4 % bis etwa 2 % Gew./Vol. und Oritavancin oder ein Salz davon in einer Konzentration von etwa 1 % Gew./Vol. vorhanden ist.

9. Pharmazeutische Zusammensetzung nach Anspruch 1, wobei die feste Form ein lyophilisiertes Pulver ist; und/oder
wobei Oritavancin als freie Base vorliegt.

10. Pharmazeutische Zusammensetzung, die Oritavancin oder ein Salz davon und Hydroxypropyl-β-cyclodextrin (HPCD) umfasst, zur Verwendung in einem Verfahren zur Behandlung einer bakteriellen Infektion, umfassend:
Verabreichen einer therapeutisch wirksamen Menge einer pharmazeutischen Zusammensetzung, die Oritavancin oder ein Salz davon, HPCD und ein wässriges Medium umfasst, an eine Person, die dies benötigt;
wobei die Verabreichung intravenös erfolgt;
wobei das HPCD und das Oritavancin oder ein Salz davon in der Zusammensetzung in einem Massenverhältnis von etwa 0,25:1 bis etwa 4:1 vorhanden sind.

11. Pharmazeutische Zusammensetzung zur Verwendung nach Anspruch 10, wobei das HPCD und das Oritavancin oder ein Salz davon in einem Verhältnis von 2:1 (Gew.-%) vorliegen.

12. Pharmazeutische Zusammensetzung zur Verwendung nach Anspruch 10 oder 11, wobei die Verabreichung abgeschlossen ist:
(a) in weniger als 3 Stunden;
(b) in weniger als 2 Stunden;
(c) in weniger als 1,5 Stunden;
(d) in weniger als 1 Stunde; oder
(e) in etwa 1 Stunde.

13. Pharmazeutische Zusammensetzung zur Verwendung nach Anspruch 10 oder 11, wobei die Zusammensetzung so konfiguriert ist, dass sie in einer Einzeldosis verabreicht wird.

14. Pharmazeutische Zusammensetzung zur Verwendung nach Anspruch 10 oder 11, wobei die Zusammensetzung ein Volumen von etwa 100 ml bis etwa 500 ml aufweist; oder
wobei die Zusammensetzung gegebenenfalls ein Volumen von etwa 250 ml aufweist.

15. Pharmazeutische Zusammensetzung zur Verwendung nach Anspruch 10 oder 11, wobei die Zusammensetzung Oritavancin oder ein Salz davon in einer Konzentration von etwa 1,2 mg/ml bis etwa 60 mg/ml aufweist; oder
wobei die Zusammensetzung gegebenenfalls Oritavancin oder ein Salz davon in einer Konzentration von etwa 2 mg/ml bis etwa 8 mg/ml aufweist.

16. Pharmazeutische Zusammensetzung zur Verwendung nach Anspruch 10 oder 11, wobei die bakterielle Infektion durch einen grampositiven Mikroorganismus verursacht wird, der ausgewählt ist aus der Gruppe bestehend aus *Staphylococcus aureus, Methicillin-resistentem Staphylococcus aureus, Methicillin-* empfindlichem *Staphylococcus aureus, Vancomycin-resistentem Staphylococcus aureus, Vancomycinintermediärem Staphylococcus aureus, Vancomycin-hetero-intermediärem Staphylococcus aureus, Streptococcus pyogenes, Streptococcus agalactiae, Streptococcus anginosus, Streptococcus intermedius, Streptococcus constellatus, Streptococcus dysgalactiae, Streptococcus dysgalactiae subsp. equisimilis, Enterococcus faecalis, Vancomycin-* resistentem *Enterococcus faecalis, Enterococcus faecium* und *Vancomycin-*resistentem *Enterococcus faecium.*

17. Pharmazeutische Zusammensetzung zur Verwendung nach Anspruch 10 oder 11, wobei die Cmax von Oritavancin in der Person nach Verabreichung etwa 100 bis etwa 300 mg/l beträgt; und/oder
wobei die AUC 0-inf von Oritavancin in der Person nach einer einzigen Verabreichung etwa 1000 bis etwa 4000 mg*h/l beträgt; und/oder
wobei die Person eine geringere Reizung der Injektionsstelle erfährt.

## Revendications

1. Composition pharmaceutique comprenant :
l'oritavancine, ou un sel de celle-ci, et l'hydroxypropyl β-cyclodextrine (HPCD) ;
dans laquelle le HPCD et l'oritavancine, ou un sel de celle-ci, sont présents dans un rapport massique d'environ 0,25:1 à environ 4:1, dans laquelle ladite composition est
a) sous forme solide qui, après reconstitution, fournit une composition pharmaceutique intraveineuse ; ou
b) dans une solution, qui est facultativement diluée, qui fournit une composition pharmaceutique intraveineuse.

2. Composition pharmaceutique selon la revendication 1, dans laquelle le HPCD et l'oritavancine, ou un sel de celle-ci, sont dans un rapport de 2:1 (poids/poids).

3. Composition pharmaceutique selon la revendication 1 ou 2, dans laquelle ladite composition pharmaceutique intraveineuse est dans un milieu aqueux.

4. Composition pharmaceutique selon la revendication 3, dans laquelle l'oritavancine, ou un sel de celle-ci, est présente dans le milieu aqueux à une concentration d'environ 1,2 mg/ml à environ 60 mg/ml ; ou facultativement
dans laquelle l'oritavancine, ou un sel de celle-ci, est présente dans le milieu aqueux à une concentration d'environ 2 mg/ml à environ 8 mg/ml.

5. Composition pharmaceutique selon la revendication 3, dans laquelle le pH de la composition est d'environ 4 à environ 8 ; ou facultativement
dans laquelle le pH de la composition est d'environ 4 à environ 6.

6. Composition pharmaceutique selon la revendication 3, dans laquelle le milieu aqueux est sélectionné dans le groupe consistant en l'eau, une solution saline normale, 5 % de dextrose dans l'eau, une solution lactée de Ringer ou des mélanges de ceux-ci.

7. Composition pharmaceutique selon la revendication 3, dans laquelle la composition est une solution :
(a) exempte de matières non dissoutes ;
(b) exempte de matières non dissoutes après 24 heures de stockage ;
(c) exempte de matières non dissoutes après 72 heures de stockage ; ou
(d) exempte de matières non dissoutes après 1 mois de stockage.

8. Composition pharmaceutique selon la revendication 3, dans laquelle le HPCD est présent à une concentration d'environ 0,2 % à environ 1 % poids/volume et l'oritavancine, ou un sel de celle-ci, est présente à une concentration d'environ 0,5 % poids/volume ; ou
dans laquelle le HPCD est présent à une concentration d'environ 0,4 % à environ 2 % poids/volume et l'oritavancine, ou un sel de celle-ci, est présente à une concentration d'environ 1 % poids/volume.

9. Composition pharmaceutique selon la revendication 1, dans laquelle la forme solide est une poudre lyophilisée ; et/ou
dans laquelle l'oritavancine est présente sous forme de base libre.

10. Composition pharmaceutique comprenant de l'oritavancine, ou un sel de celle-ci, et de l'hydroxypropyl β-cyclodextrine (HPCD) destinée à être utilisée dans un procédé de traitement d'une infection bactérienne comprenant :
l'administration à un sujet en ayant besoin d'une quantité thérapeutiquement efficace d'une composition pharmaceutique comprenant de l'oritavancine, ou un sel de celle-ci, du HPCD, et un milieu aqueux ;
dans laquelle l'administration est intraveineuse ;
dans laquelle le HPCD et l'oritavancine, ou un sel de celle-ci, sont présents dans la composition dans un rapport massique d'environ 0,25:1 à environ 4:1.

11. Composition pharmaceutique pour une utilisation selon la revendication 10, dans laquelle le HPCD et l'oritavancine, ou un sel de celle-ci, sont dans un rapport de 2:1 (poids/poids).

12. Composition pharmaceutique pour une utilisation selon la revendication 10 ou 11, dans laquelle l'administration est accomplie :
(a) en moins de 3 heures ;
(b) en moins de 2 heures ;
(c) en moins de 1,5 heure ;
(d) en moins de 1 heure ; ou
(e) en 1 heure environ.

13. Composition pharmaceutique pour une utilisation selon la revendication 10 ou 11, dans laquelle la composition est configurée pour être administrée en une dose unique.

14. Composition pharmaceutique pour une utilisation selon la revendication 10 ou 11, dans laquelle la composition présente un volume d'environ 100 ml à environ 500 ml ; ou facultativement
dans laquelle la composition présente un volume d'environ 250 ml.

15. Composition pharmaceutique pour une utilisation selon la revendication 10 ou 11, dans laquelle la composition présente de l'oritavancine, ou un sel de celle-ci, à une concentration d'environ 1,2 mg/ml à environ 60 mg/ml ; ou facultativement
dans laquelle la composition présente de l'oritavancine, ou un sel de celle-ci, à une concentration d'environ 2 mg/ml à environ 8 mg/ml.

16. Composition pharmaceutique pour une utilisation selon la revendication 10 ou 11, dans laquelle l'infection bactérienne est provoquée par un micro-organisme Gram positif sélectionné dans le groupe consistant en *Staphylococcus aureus, Staphylococcus aureus résistant à la méthicilline, Staphylococcus aureus* sensible *à la méthicilline, Staphylococcus aureus résistant à la vancomycine, Staphylococcus aureus intermédiaire à la vancomycine, Staphylococcus aureus hétéro-intermédiaire à la vancomycine, Streptococcus pyogenes, Streptococcus agalactiae, Streptococcus anginosus, Streptococcus intermedius, Streptococcus constellatus, Streptococcus dysgalactiae, Streptococcus dysgalactiae subsp. equisimilis, Enterococcus faecalis, Enterococcus faecalis* résistant *à la vancomycine, Enterococcus faecium* et *Enterococcus faecium* résistant *à la vancomycine.*

17. Composition pharmaceutique pour une utilisation selon la revendication 10 ou 11, dans laquelle la Cmax de l'oritavancine chez le sujet après administration est d'environ 100 à environ 300 mg/l ; et/ou
dans laquelle l'AUC 0-inf de l'oritavancine chez le sujet après une administration unique est d'environ 1 000 à environ 4 000 mg*h/I ; et/ou
dans laquelle le sujet subit une irritation réduite au site d'injection.
